(19) **Europäisches Patentamt** / **European Patent Office** / **Office européen des brevets**

(11) **EP 4 440 526 B1**

(12) **EUROPEAN PATENT SPECIFICATION**

(45) Date of publication and mention of the grant of the patent:
**01.04.2026   Bulletin 2026/14**

(21) Application number: **21824566.0**

(22) Date of filing: **03.12.2021**

(51) International Patent Classification (IPC):
*A61F 13/49* (2006.01)      *A61F 13/494* (2006.01)
*A61F 13/495* (2006.01)      *A61F 13/505* (2006.01)
*A61F 13/551* (2006.01)

(52) Cooperative Patent Classification (CPC):
**A61F 13/49011; A61F 13/49466; A61F 13/49473;**
**A61F 13/495; A61F 13/505; A61F 13/55105;**
A61F 2013/4948

(86) International application number:
**PCT/EP2021/084178**

(87) International publication number:
**WO 2023/099012 (08.06.2023 Gazette 2023/23)**

(54) **ABSORBENT ARTICLE COMPRISING A FAECES POCKET**

ABSORBIERENDER ARTIKEL MIT EINER FÄKALIENTASCHE

ARTICLE ABSORBANT COMPRENANT UNE POCHE POUR MATIÈRES FÉCALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.10.2024   Bulletin 2024/41**

(73) Proprietor: **Essity Hygiene and Health Aktiebolag**
**405 03 Göteborg (SE)**

(72) Inventors:
 • **BADERSTEDT, Julia**
 **431 64 Mölndal (SE)**
 • **VARTIAINEN, Kent**
 **443 92 Lerum (SE)**
 • **SILLERSTRÖM, Filip**
 **435 35 Mölnlycke (SE)**
 • **TOURNIER, Paul**
 **405 03 Göteborg (SE)**
 • **HAMMARROTH, Susanna**
 **405 03 Göteborg (SE)**

(74) Representative: **Hoffmann Eitle**
**Patent- und Rechtsanwälte PartmbB**
**Arabellastraße 30**
**81925 München (DE)**

(56) References cited:
**WO-A1-2016/159978      US-B2- 10 470 943**

## Description

### TECHNICAL FIELD

The present disclosure relates to an absorbent article comprising a main portion having a front region, a rear region and a crotch region located between the front and rear regions, the front region and the rear region being adapted to form part of a waist edge circumfering the waist of a user when the article is worn.

### BACKGROUND

In the field of absorbent articles, such as absorbent diapers and pant-type articles, there is a general desire to provide absorbent articles which are perceived as comfortable as well as providing satisfactory absorbency and leakage security.

For providing leakage security, various features have been proposed, such as waist elastics, leg elastics, leg barriers and/or waist barriers, for reducing the risk of leakage of bodily fluids from the zones around the legs or the waist of the wearer. In particular, for hindering leakage of faeces from the waist region towards the back of the wearer, it has been proposed to provide the absorbent article with a faeces pocket, arranged in the rear region of the absorbent article.

Adding features for increasing leakage security of an article often imply a tighter fit of the article around the wearer and/or a thicker product, both of which might impact the wearer's perceived comfort and breathability of the article. As such, there is a desire to provide for satisfactory leakage security while achieving a satisfactory comfort and perceived breathability of the article.

In addition, there is a continuing need for improvements in the field in particular with regard to reduction of material consumption as well as cost efficiency. This is in particular the case when manufacturing disposable absorbent articles. There is also an increasing concern from an environmental point of view that the amount of material used in disposable absorbent articles is kept to a minimum. Thus, there is in general a desire for an absorbent article, such as a disposable absorbent article, to be manufactured in a cost efficient and material saving manner.

Hence, it is desired to provide an absorbent article, such as a disposable absorbent article, which constitutes an improvement or a useful alternative in view of one or more of the above-mentioned needs.

US 10 470 943 B2 discloses an absorbent article that can include a chassis including an absorbent body. The chassis can include a body facing surface. The absorbent article can also include a waist containment member. The waist containment member can include a proximal portion coupled to the body facing surface of the chassis and a distal portion that includes a distal edge. The proximal portion includes proximal portion elastic members and the distal portion can include distal portion elastic members. The distal portion can be free to move with respect to the chassis when the absorbent article is in a relaxed configuration.

### SUMMARY

One or more of the above objects is achieved with an absorbent article according to the present invention as defined in appended independent claim 1. Further embodiments are set out in the dependent claims and in the following description.

As such, there is provided an absorbent article comprising a main portion extending in a length direction along a central length axis between a front edge and a rear edge of the absorbent article, and in a width direction along a central width axis, perpendicular to said central length axis between a pair of side edges of the absorbent article, and extending in a height direction perpendicular to the width and length direction.

The main portion defines a main inner surface extending primarily along the width and length directions to be directed towards the wearer when the article is worn, and a main outer surface extending primarily along said width and length directions to be directed away from the wearer when the article is worn.

The main portion defines a front region comprising said front edge, a rear region comprising said rear edge, and a crotch region located between the front and rear regions. The front region and the rear region are adapted such that the front edge and the rear edge form part of a waist edge circumfering the waist of a user when the article is worn.

The article further comprises a pocket member extending along a pocket length in the length direction between a pocket rear edge and a pocket front edge, and along a pocket width in the width direction between a pair of pocket side edges. The pocket length referred to herein is a maximum pocket length as measured in parallel to the length direction between the pocket rear edge and the pocket front edge. The pocket width referred to herein is a maximum pocket width as measured in parallel to the width direction between the pocket side edges.

The pocket member is attached to the rear region of the main portion by a rear attachment element extending along the pocket rear edge, and side attachment elements extending along the pocket side edges, such that the attachment elements form a closed faeces boundary between the pocket member and the main portion, with an opening at the pocket front edge towards the crotch region of the main portion.

The pocket member comprises a plurality of elastic members providing the pocket member with elastic characteristics along the width direction, the elastic members being distributed over the pocket length of the pocket member so as to form a first elastic zone extending from the pocket rear edge along the length direction, a second elastic zone extending from the pocket front edge along the length direction, and an intermediate zone extending in the length direction from the first elastic zone to the second elastic zone and over at least 20% of the pocket length, and an average distribution of the elastic members in the intermediate zone is less than 0.5 elastic members per cm as seen in the length direction, and an average distribution of the elastic members in each of the first and second elastic zone is greater than in the intermediate zone, as seen in the length direction.

Thus, the first elastic zone extends from the pocket rear edge and along the length direction in a direction towards the pocket front edge. Similarly, the second elastic zone extends from the pocket front edge and along the length direction in a direction towards the pocket rear edge. The intermediate zone extends along the length direction between the first elastic zone and the second elastic zone.

Thus, the elastic members are distributed over the pocket length of the pocket member so as to divide said pocket length into the first elastic zone, the second elastic zone, and the intermediate zone.

The absorbent articles referred to herein are wearable absorbent articles, for example in the form of open diapers, incontinence garments, and the like, as well as absorbent inserts which are worn inside a support garment, such as a support pant or ordinary underwear. The articles are used to absorb, distribute and store various types of body exudates while providing a high level of comfort and a sense of dryness to the wearer during use.

The absorbent article may be a disposable article, i.e. an article which is discarded after a limited number of uses, for example after only one use, rather than washed and used again.

The absorbent article may be a hybrid article, i.e. an article which comprises a disposable portion which is discarded after a limited number of uses, and a reusable portion which may be washed and used again and which is thus intended for a greater number of uses.

The absorbent article as proposed herein provides a pocket member comprising elastic members, wherein the distribution of the elastic members enables satisfactory function of the pocket member for accommodating bodily exudates and contributes to providing increased comfort for the wearer.

As proposed herein, the first elastic zone and the second elastic zone of the pocket member will have a denser average distribution of elastic members than the intermediate zone.

As such, the first elastic zone may contribute to the waist elastic of the absorbent article, and hence to the fit and comfort of the absorbent article. As will be further discussed in the below, the provision of the first elastic zone of the pocket member at a location where it may contribute to the waist elastic of the absorbent article enables variants which may be advantageous in terms of manufacture, use of material and breathability of the article.

The second elastic zone may contribute to the function of the pocket member in that the second elastic zone may urge the pocket member towards the back of the user when the article is worn, hence ensuring that the faeces pocket formed by the pocket member will assume an open state with the opening of the pocket being directed towards the crotch region of the absorbent article.

Further, the intermediate zone, wherein an average distribution of the elastic members in the intermediate zone is less than 0.5 elastic members per cm as seen in the length direction, may provide a volume to the pocket formed by the pocket member for receiving the bodily exudates. Further, the intermediate zone may provide for satisfactory breathability of the pocket member, thus improving the comfort for the wearer of the article.

By the disclosed pocket member, with the first and second elastic zones interrupted by the intermediate zone, the opening of the pocket member during wear of the article is ensured without requiring the pocket member to be arranged so as to cooperate with other members such as leakage barriers and/or elastic side portions of the article. As such, versatility and production advantages may be provided. Also, superfluous addition of several material layers along the height direction of the article may be avoided, i.e. the pocket member may be arranged with less perceived addition to the thickness of the product than with outer solutions.

Further, this provides for that the sheet material used in the pocket member may be spared from excessive strain, and so relatively thin and/or low weight sheet materials may be used, contributing to increased perceived softness and breathability as well as to reduced use of raw material.

The intermediate zone extends over at least 20% of the pocket length. For example, the intermediate zone may extend over at least 25% of the pocket length. Optionally, the intermediate zone may extend over at least 30% of the pocket length.

Optionally, the intermediate zone extends in the length direction over no more than 80% of the pocket length.

Optionally, each one out of the first and second elastic zones extends over a length being less than the length of the intermediate zone as seen in the length direction. As such, the intermediate zone may extend over at least one third of the pocket length.

Optionally, each one out of the first and second elastic zones extends over at least 10% of the pocket length as seen in the length direction.

The length extension of the first elastic zone may be deemed to be the length as measured from the pocket rear edge and so as to include, but not to extend beyond, the elastic member farthest from the pocket rear edge and which is deemed to form part of the first elastic zone.

The length extension of the second elastic zone may be deemed to be the length as measured from the pocket front edge and so as to include, but not to extend beyond, the elastic member farthest from the pocket front edge and which is deemed to form part of the second elastic zone.

Optionally, the first and/or second elastic zone, and/or the intermediate zone extend substantially in parallel to the width direction. As such, the first and second elastic zone and the intermediate zone may be essentially rectangular.

Optionally, the elastic members extend substantially in parallel to the width direction.

As mentioned in the above, the elastic members provide the pocket member with elastic characteristics along the width direction thereof. As such, the pocket member may be essentially inelastic in the length direction.

The elastic members may be arranged such that the first and/or second elastic zone(s) are continuous as seen along the width direction, for example such that the elastic members extend continuously over the pocket width of the pocket member.

Optionally, the pocket member is free from elastic members in the intermediate zone. That the pocket member is free from elastic members in the intermediate zone, which as mentioned in the above, extends in the length direction, along at least 20% of the pocket length, may provide for satisfactory volume of the pocket being formed by the pocket member as well as enhance the breathability of the pocket member. For example, as set out in the above, the pocket member may be free from elastic members in the intermediate zone, the intermediate zone extending over at least 25%, for example over at least 30% or at least 1/3 of the pocket length of the pocket member.

Optionally, in each of the first and/or second elastic zone, an average distribution of the elastic members is greater than 0.5 members/cm. For example, the average distribution may be greater than 1 member/cm, such as within the range from 1 to 3 members/cm. For example, the average distribution may be 2 members per centimeter.

Optionally, at least one out of the first and/or second elastic zone comprises a plurality of elastic members, wherein each elastic member is spaced from neighbouring elastic members as seen in the length direction.

Optionally, both the first and the second elastic zone comprises a plurality of elastic members, wherein each elastic member is spaced from neighbouring elastic members as seen in the length direction.

Optionally, in the first elastic zone, the elastic members may be regularly distributed as seen along the length of the pocket member. In other words, a distance along the length direction between two neighbouring elastic members is the same for each pair of neighbouring elastic members throughout the first elastic zone.

Optionally, in the second elastic zone, the elastic members may be regularly distributed as seen along the length of the pocket member. In other words, a distance along the length direction L between two neighbouring elastic members is the same for each pair of neighbouring elastic members throughout the second elastic zone.

The neighbouring elastic members may be arranged in parallel to each other.

Optionally, in the first and/or second elastic zone a distance between two neighbouring elastic members is at least 3 mm, such as within the range from 3 to 7 mm, as seen in the length direction. For example, the distance between two neighbouring elastic members may be 5 mm.

For example, in the first and/or elastic zone, for each pair of neighbouring elastic members the distance between the neigbouring elastic members may be the same, and be at least 3 mm, such as within the range from 3 to 7 mm, such as 5 mm.

The distance between the elastic members may be relevant for the breathability of the pocket member.

However, the distance between the elastic members is also relevant for the behaviour of the pocket member when the absorbent article is in use. The distance between the elastic members may be adjusted for setting a desired amount of pressure from the article towards the wearers body. As such, the distance between the elastic members in the first elastic region may influence the pressure from the article around the waist of the wearer, whereas the distance between the elastic members in the second elastic region may influence the pressure with which the open end of the pocket will strive towards the wearer's back. For example, a relatively large distance between the elastic members reduces the pressure towards the wearer's back.

Moreover, the type, elasticity, thickness and number of elastic members may influence the above-mentioned pressures.

Optionally, the first and/or second elastic zone each comprises no more than seven elastic members. For example, the first and/or second elastic zone may each comprise no more than five elastic members. For example, the first and/or second elastic zone may comprise three elastic members.

Optionally, the elastic members are elastic threads.

The elastic members may be arranged in parallel to each other, for example along the width direction.

The thickness of the elastic threads may be selected considering that relatively thinner threads may reduce the pressure towards the back of the wearer, though requirements for strength, durability and/or elasticity must also be fulfilled. For example, selecting relatively thinner threads with relative greater distance between neighbouring threads may contribute to reducing the pressure towards the wearer's back, i.e. reducing the risk of the elastic zone of the pocket member "cutting in" towards the wearer's body in an uncomfortable manner, as compared to variants with relatively coarser threads or threads being positioned more densely.

For example, the elastic members may have a thickness within the range from 400 to 1100 dtex.

Optionally, one or more, preferably all of the elastic members of the first and second elastic zones have an elasticity within the range from 70% to 250%, such as 100% to 250%.

"Elasticity" as used herein refers to the elongation in the elastic material which is expressed as a percentage of the length of the unstretched material compared to the stretched thread.

The elongation is calculated according to the formula below and state the accuracy of the final results of the strain/elongation/tension with integer in %.

$L_2$ = Length of the fully stretched elastic threads
$L_1$ = Length of the unstretched elastic threads (mm).
$E$ = Strain/elongation/tension in the elastic threads (%).

$$E = \frac{L_2 - L_1}{L_1} \times 100$$

For determination of the length of the fully stretched elastic thread, the thread is arranged so as hang vertically under the load of a 100 g weight.

Optionally, the first elastic zone is arranged adjacent the rear edge of the main portion. As such, the first elastic zone may be arranged so as to form a rear waist elastic, contributing to the elasticity of the rear region of the main portion around the waist of the user when the article is worn.

Thus, the first elastic zone may be arranged to extend along the waist edge of the main portion. As such, the first elastic zone may be arranged to extend essentially in parallel with the waist edge.

Advantageously, the pocket rear edge may be arranged in parallel to the rear edge of the main portion.

For example, the pocket rear edge may be arranged along the rear edge of the main portion such that there is no distance in the length direction between the pocket rear edge and the rear edge of the main portion.

In other variants, the pocket rear edge may be arranged to extend along the rear edge with a distance as seen in the length direction from the rear edge. In this case, the main portion may extend beyond the pocket member to form the rear edge of the article. As such, this may contribute to a soft waist edge being formed by the main portion extending beyond the pocket member.

Optionally, the rear region of the main portion may be free from elastic members providing elasticity in the width direction at least over a width extension corresponding to the extension as seen in the width direction of the first elastic zone. As such, the elasticity in the width direction of the rear region of the article as a whole and over the width extension of the first elastic zone is provided by the first elastic zone only.

Further, the rear region of the main portion may be free from any elastic members providing elasticity in the width direction. As such, the elasticity in the width direction of the rear region of the article is provided by the first elastic zone. Thus, the first elastic zone forms the rear waist elastics of the article as a whole. The first elastic zone may hence replace any other rear waist feature provided to the article. The first elastic region acting as a rear waist elastic may hence be the only waist elastic provided adjacent the rear edge of the main portion of the article.

Optionally, the front region of the main portion may comprise front waist elastic members. In another option, the main portion may be free from elastic members providing elasticity in the width direction. As such, the first elastic region acting as a rear waist elastic is the only waist elastic provided adjacent the front or rear edge of the main portion of the article. This enables selecting materials for the main portion and manufacturing the main portion without consideration of addition of such elastic members.

The first elastic region acting as a rear waist elastic provides for an efficient and material-saving manner of providing a waist elastic.

Optionally, the rear attachment element may at least partly overlap the first elastic zone as seen along the height direction. Accordingly, the elasticity provided by the first elastic zone may effectively elasticize the main portion. Optionally, the rear attachment element may completely overlap the first elastic zone as seen along the height direction. Thus, the elasticity provided by the first elastic zone may even more efficiently elasticize the main portion.

For example, the rear attachment element may essentially coincide with the first elastic zone, as seen along the height direction.

The pocket member may comprise a sheet material. As such, the sheet material may be provided with the elastic members, i.e. the elastic threads. For example, the elastic members may be attached to the sheet material by means of an adhesive.

The sheet material may be selected so as to provide satisfactory breathability to the pocket member, while still ensuring satisfactory retaining properties for the bodily exudates.

Optionally, the sheet material may be a nonwoven material.

Optionally, the pocket member comprises two layers of sheet material. As such, the elastic members, such as elastic threads, may be positioned between the two layers of sheet material.

Optionally, the two layers of sheet material are formed by a continuous piece of sheet material being folded around the elastic members, and joined by a joint to form the pocket member.

In this case, the joint may be located in the intermediate zone of the pocket member. As such, any risk of the joint adversely affecting the elastic properties of the pocket member in the first or second elastic zone is avoided.

Preferably, the joint may be located on a main portion facing side of the pocket member. As such, it will not contact the wearer's skin during use of the article.

Optionally, the joint may be an adhesive joint. As such, the joint may join two edge portions of the folded sheet material to each other in a partly overlapping relationship as seen in the height direction.

Optionally, the joint may be a continuous joint.

Alternatively, the joint may be a non-continuous joint.

Optionally, the joint may form a joint length extension along the length direction of the article, and a joint width extension along the width direction of the article. The joint width extension may be greater than the joint length extension...

Optionally, the joint length extension is at least 2 mm. Optionally, the joint length extension is less than 5 mm.

The joint width extension may correspond the entire pocket width of the pocket member.

Optionally, the intermediate zone may consist of the sheet material only.

Optionally, when the pocket member comprises a folded sheet material with a joint as described in the above, the intermediate zone may consist of the two layers of sheet material and the joint.

As such, the intermediate zone is arranged so as to be free from components adversely affecting the breathability of the intermediate zone. I.e. the breathability of the intermediate zone will primarily be dependent on the breathability of the sheet material per se.

Optionally, out of a total planar area of the pocket member, at least 75% comprises sheet material only. That is, if considering the pocket member as laid out in the length and width direction with the elastic properties relaxed, as seen from the height direction, at least 75% of the area of the pocket member comprises sheet material only. Any area occupied by elastic members, joints or other additional elements in the pocket member are hence removed from the area considered to comprise sheet material only. (However, the area covered by the attachment elements are not removed from the area considered to comprise the sheet material only. Areas comprising one or more layers of sheet material, such as two layers of sheet material, are considered to comprise sheet material only.)

In a similar vein, out of a total planar area of the intermediate zone, at least 90 % may comprise sheet material only.

The sheet material may have a basis weight within the range from 10 gsm to 60 gsm. For example, the sheet material may have a basis weight within the range from 10 gsm to 30 gsm.

Optionally, the sheet material may be a nonwoven having a basis weight within the range from 10 gsm to 60 gsm. For example, the sheet material may be a nonwoven having a basis weight within the range from 10 gsm to 30 gsm. As such, a sheet material having satisfactory breathability may be provided. For example, the sheet material being a nonwoven and having a basis weight as indicated in the above may have an air permeability and a Material Water Vapor Transmission rate as indicated below.

Optionally, the sheet material may have an air permeability of at least 1000 l/m2/s. For example, the sheet material may have an air permeability of at least 2000 l/m2/s.

"Air permeability" as used herein is measured according to Edana standard Method WSP 70.1.

Optionally, the sheet material may have a Material Water Vapor Transmission rate of at least 2000 g/m2/24h. For example, the sheet material may have a Material Water Vapor Transmission rate of at least 4000 g/m2/24 h.

"Material Water Vapor Transmission rate" as used herein is measured according to ASTM "-96.

For example, the sheet material may have an air permeability of at least 1000 l/m2/s and a Material Water Transmission rate of at least 2000 g/m2/24h. Such a sheet material may preferably be a nonwoven material.

For example, the sheet material may have an air permeability of at least 2000 l/m2/s and a Material Water Transmission rate of at least 2000 g/m2/24h. Such a sheet material may preferably be a nonwoven material.

For example, the sheet material may have an air permeability of at least 2000 l/m2/s and a Material Water Transmission rate of at least 4000 g/m2/24h. Such a sheet material may preferably be a nonwoven material.

For example, the sheet material may be a nonwoven material comprising fibres composed out of one or more components. Suitable components may be for example PP or PE. As such, the fibers may be for example PP-PE fibers or PP-PP fibers.

The nonwoven material may for example be spunbonded or carded.

The main portion may comprise an absorbent core extending at least in the crotch region of the main portion.

Further, the main portion may comprise a top sheet forming at least a part of the inner main surface of the main portion, and a back sheet forming at least a part of the outer main surface of the main portion.

For example, the back sheet may extend continuously over the main portion, thus forming a continuous outer main surface of the main portion.

For example, the top sheet may extend continuously over the main portion, thus forming a continuous inner main surface of the main portion. In another example, the top sheet may extend over a part of the main portion comprising the absorbent core, such as over a central part of the main portion as seen along the width direction. One or more additional sheet materials, such as e.g. a leakage barrier forming material and/or a leg gasket forming material may be joined to the top sheet such that said top sheet and at least portions of said one or more additional sheet materials together form the inner main surface of the main portion.

The absorbent core is arranged between the top sheet and the back sheet. As such, it is understood that the back sheet and top sheet forming at least part of the inner main surface and the outer main surface of the main portion, respectively, will face generally in opposite directions along the height direction of the main portion.

The main portion may be such that the top sheet and back sheet extend outside of the absorbent core and are joined to each other in the front and/or in the rear region of the main portion.

Optionally, the pocket member may be arranged with no overlap in relation to the absorbent core as seen in the height direction i.e. the pocket member is arranged in a non-overlapping relationship with the absorbent core as seen in the height direction. The lack of overlap contributes to avoiding unnecessary layers of materials on top of each other in the height direction, which may impact the breathability of the absorbent article.

Optionally, the pocket member is arranged in relation to the absorbent core such that there is a distance in the length direction between the pocket front edge of the pocket member and the absorbent core. Thus, such a distance will extend between the pocket front edge and a rearmost edge of the absorbent core.

Providing for a distance between the pocket front edge and the absorbent core facilitates the opening of the pocket, i.e. forming of a gap between the inner main surface of the main portion and the front edge of the pocket member such that the faeces may enter the pocket easily. Further, as the absorbent core may expand in the height direction upon absorbing fluid, the distance prevents the absorbent core from blocking the pocket opening.

Optionally, the pocket member is arranged in relation to the absorbent core such that a distance in the length direction between the front edge of the pocket member and the absorbent core is 5 mm or more. For example, the distance may be 7 mm or more. For example, the distance may be 12 mm or more. For example, the distance may be within the range from 5 to 12 mm, such as within the range from 7 to 12 mm. For example, the distance may be 10 mm.

Optionally, the absorbent core may comprise regions as seen in the width and length direction comprising varying amounts of absorbent material. For example, the absorbent core may be provided with less absorbent regions or lines extending generally along the length direction. The less absorbent regions or lines may comprise absorbent material in a lesser amount than the surrounding regions of the absorbent core. In some cases, the less absorbent regions or lines may comprise no absorbent materials. In some variants, such less absorbent regions or lines display a lesser height as seen along the height direction than the surrounding regions of the absorbent core. Less absorbent regions or lines may assist in forming the core to a shape conforming to the wearer during use of the article. With the pocket member as proposed herein, less absorbent regions or lines arranged at least in a rear portion of the absorbent core may assist in forming the core so as to contribute to the function of the pocket, and/or to guide faeces material towards the pocket opening.

Optionally, the absorbent core may display a front cut-out as seen from a front edge of the absorbent core. Such a front cut-out may be shaped so as to form a space for the belly button of a wearer, this being of particular interest for baby diapers of relatively small sizes as used for very young babies.

Optionally, the top sheet and the back sheet are joined in the rear region of the main portion, and the pocket member is arranged in an overlapping relationship with the joined top sheet and back sheet in the rear region of the main portion, such that the joined top sheet and back sheet completely overlaps the pocket member as seen in the height direction. As such, the rear region of the article may be made relatively thin and soft.

Optionally, the pocket member is attached to said inner main surface of said main portion by said rear and side attachment elements. As such, the pocket member may be attached directly to the inner main surface via the attachment elements. Accordingly, the pocket member does not require fastening to any additional members, such as leakage barriers or the like. This provides for easier manufacture, and also enables constructions which only require a limited number of superposed layers in the article.

The pocket member may form a first surface and a second surface opposite to the first surface. Optionally, the pocket member is arranged with the first surface forming a wearer facing surface, and the second surface forming a main portion facing surface, wherein the attachment elements attach the second surface to the main portion. Accordingly, the pocket member is arranged to the main portion in an unfolded state. This provides for a relatively thin article, by avoiding layers of material being superposed over each other, as well as for easy manufacture. Further, the attachment elements may attach the second surface to the main inner surface of the main portion. Again, this enables forming a relatively thin product with relatively few layers of material superposed and/or attached to one another.

Optionally, the attachment elements may extend along substantially the entire pocket rear edge, first and second side edges, respectively.

Optionally, each one of the side attachment elements may be arranged outward of said absorbent core, as seen along the width direction. Thus, the opening of the pocket may extend over the entire width of the core.

Optionally, the said rear and side attachment elements are arranged in an area of said main portion comprising a joined topsheet and backsheet. Thus, the rear and side attachment element may be arranged in an area of the main portion where the joined topsheet and backsheet extends beyond the core. As such, the pocket member may be provided where the main portion is relatively thin and soft.

Optionally, the rear and side attachment elements comprise an adhesive.

Optionallly, the rear and side attachment elements cover no more than 60% of a total planar surface area A of the pocket member. For example, the rear and side attachment elements may cover no more than 50% of a total planar surface area of the pocket member. The total planar surface area of the pocket member is to be determined as indicated in the above, with the pocket member completely extended along the length and width directions, i.e. with the elasticity of the elastic members removed.

Optionally, said absorbent article further comprises a pair of leakage barriers.

With a "leakage barrier" as used herein it is referred to a structure out of which at least a portion is intended to be raised during use of the article, so as to form a leakage barrier to hinder leakage at the wearer's legs. Such leakage barriers may also be referred to as raised elastic members, and are commonly known as standing gathers.

Each leakage barrier may be joined to the inner main surface of the main portion along a leakage barrier joint extending in said length direction at least along the crotch portion. The leakage barrier extends from said leakage barrier joint in the height and/or width extension along a leakage barrier width to a leakage barrier edge.

A leakage barrier active region may be provided at least in said crotch portion of the main portion, wherein said leakage barrier extends freely from said leakage barrier joint along said leakage barrier width to a free leakage barrier edge. As such, the leakage barrier active region is a region of the leakage barrier where the leakage barrier extends freely along the leakage barrier width so as to enable the leakage barrier to rise from the inner main surface of the main portion and assume an active position in which the leakage barrier forms a barrier to sideway leakage at the legs of the wearer when the article is worn.

Optionally, the free leakage barrier edge comprises a leakage barrier elastic member to provide elasticity generally along the length direction of said free leakage barrier edge. As such, in the active region, the leakage barrier may be provided with a leakage barrier elastic member so as to enable a tight fit around the legs of the wearer.

Leakage barriers as described in the above may be arranged along both longitudinal sides of the main portion to provide a leakage barrier at each leg of the wearer as well as in improved fit. As such, the leakage barriers may complement the pocket member whose purpose is to hinder leakage and improve fit at the rear region of the article. However, for the pocket member proposed herein it is desired that the function of the leakage barrier members do not interfere with the function of the pocket member. For example, it is desired that the expansion of the pocket member, its location or volume, is generally unaffected by e.g. leakage barrier elastic members. Thus, as will be proposed in the below, it is desired that the pocket member is arranged in a manner which allows the combination of the pocket member with the leakage barriers in an article without unnecessarily connecting these elements. The features proposed in the below generally allows for easy production and again for relatively few material layers to be arranged on top of each other, thus contributing to comfort and breathability of the product.

Optionally, the pocket member is arranged in a non-overlapping relationship with the leakage barrier elastic member as seen in the height direction of the article.

As such, there is a distance in the length direction between the pocket front edge and the leakage barrier elastic member. This distance may for example be at least 10 mm.

Optionally, the pocket member is arranged in a non-overlapping relationship with the leakage barrier active region, as seen in the height direction H.

Optionally, there is a distance in the length direction L between the pocket front edge and said leakage barrier active region. The leakage barrier active region, as explained in the above, may be provided at least in said crotch portion of the main portion, and is the region of the leakage barrier wherein said leakage barrier extends freely from said leakage barrier joint along said leakage barrier width to a free leakage barrier edge.

Optionally, a leakage barrier fastening region may be provided, wherein the leakage barrier is fastened to the inner main surface of the main portion at a leakage barrier fastening element between the leakage barrier joint and the leakage barrier edge, and/or at the leakage barrier edge, such that the leakage barrier extends freely from the leakage barrier fastening element in the height and/or width extension along a leakage barrier unfastened width being less than the leakage barrier width. As such, it is understood that in the leakage barrier fastening region the leakage barrier does not extend freely over the entire leakage barrier width as available in the active region.

Optionally, the pocket member is arranged in at least a partly overlapping relationship with the leakage barrier fastening region, as seen in the height direction H.

Optionally, in said overlapping portion of the leakage barrier fastening region, said leakage barrier unfastened width is greater than 50% of said leakage barrier width.

Optionally, the pocket member is arranged in a partly overlapping relationship with said leakage barrier fastening region wherein said leakage barrier unfastened width is less than 20% of said leakage barrier width, such as less than 10% of said leakage barrier width, for example 0.

Optionally, the side attachment elements are arranged outwardly of the leakage barrier joints as seen in the width direction of the article. As such, the pocket opening may extend over the leakage barrier, to the benefit of the function of the combination of the leakage barriers and the pocket member.

Optionally, the attachment elements are arranged in a non-overlapping relationship with said leakage barriers, as seen in the height direction of the article.

Optionally, the attachment elements are arranged to the inner main surface, such as to said inner main surface only.

Thus, for example, the attachment elements may be arranged so as to coincide with portions of the main portion comprising only the joined top sheet and back sheet, as seen in the height direction. Again, this provides for a relatively thin and breathable article.

Optionally, the absorbent article comprises side portions, such as elastic side portions, attached to said main portion and extending in the width direction on each side of the rear region of the main portion. Side portions, such as elastic side portions, may in some variants be arranged to be fastened to the front region of the main portion, e.g. using suitable fastening means. The side portions may form part of a waist edge of the article, which together with the waist edge of the front and/or rear portion of the main portion may surround the waist of the wearer when the article is worn. Notably, with the term "main portion" as used herein, any elastic side portions do not form part of the main portion.

When the absorbent article comprises side portions, such as elastic side portions, the pocket member is arranged in a non-overlapping relationship with the side portions as seen in the height direction of the article.

When the absorbent article comprises elastic side portions and leakage barrier members as described in the above, each side attachment element may be arranged at the main portion between the elastic side portion and the leakage barrier joint.

Optionally, the disposable article has an article length along the length direction, wherein a pocket length proportion being the pocket length / the article length is within the range from 5% to 20 %.

Optionally, the pocket length is within the range from 20 to 60 mm.

As mentioned in the above, the absorbent article may be disposable absorbent article.

Alternatively, the absorbent article comprises a disposable portion and a reusable portion. In this case, the pocket member may for example form part of the disposable portion. For example, the main portion carrying the pocket member and optionally leakage barriers may form part of a disposable portion.

As set out in the above, the absorbent article according to the first aspect, comprising a pocket member having a first elastic region, a second elastic region, and an intermediate region may provide specific advantages in that a well-functioning pocket may be formed and may be combined with other features providing additional advantages, for example in terms of comfort and breathability of the article.

However, some of the advantages described in the above in relation to different features which may optionally be added to the first aspect, may also be available in variants of articles not comprising the specific pocket member as set out in the first aspect.

For example, the feature that the pocket member is arranged in a non-overlapping relationship with the absorbent core as seen in the height direction may contribute to the comfort and breathability of the article, as well ensuring that the pocket opening is not blocked by an expanded core, also in variants of articles wherein the pocket member per se has a different distribution of the elastic members than what is set out in the first aspect.

As such, in a first alternative aspect not forming part of the invention as defined in the appended claims, there may be provided an absorbent article comprising a main portion extending in a length direction along a central length axis between a front edge and a rear edge of the absorbent article, and in a width direction along a central width axis, perpendicular to the central length axis between a pair of side edges of the absorbent article, and extending in a height direction perpendicular to the width and length directions, the main portion defining a main inner surface extending primarily along the width and length directions to be directed towards the wearer when the article is worn, and a main outer surface extending primarily along the width and length directions to be directed away from the wearer when the article is worn; the main portion defining a front region comprising the front edge and a rear region comprising the rear edge, and a crotch region located between the front and rear regions, the front region and the rear region being adapted such that the front edge and the rear edge form part of a waist edge circumfering the waist of a user when the article is worn, and the main portion comprises an absorbent core extending at least in the crotch region of the main portion and the pocket member is arranged in a non-overlapping relationship with the absorbent core as seen in the height direction.

This first alternative aspect may be combined with one or more of the features as set out in the above in relation to the first aspect, so as to obtain similar advantages.

In another example, the feature that each one out of the side attachment elements are arranged outwardly of the respective leakage barrier joint as seen in the width direction, may contribute to the comfort and breathability of the article, as well ensuring the function of the pocket, also in variants of articles wherein the pocket member per se has a different distribution of the elastic members than what is set out in the first aspect.

As such, in a second alternative aspect not forming part of the invention as defined in the appended claims, there is provided an absorbent article comprising a main portion extending in a length direction along a central length axis between a front edge and a rear edge of the absorbent article, and in a width direction along a central width axis, perpendicular to the central length axis between a pair of side edges of the absorbent

article, and extending in a height direction perpendicular to the width and length directions, the main portion defining a main inner surface extending primarily along the width and

length directions to be directed towards the wearer when the article is worn, and a main outer surface extending primarily along the width and length directions to be directed away from the wearer when the article is worn;

the main portion defining a front region comprising the front edge and a rear region comprising the rear edge, and a crotch region located between the front and rear regions, the front region and the rear region being adapted such that the front edge and t the edge form part of a waist edge circumfering the waist of a user when the article is worn, wherein the absorbent article further comprises a pair of leakage barriers, each leakage barrier being joined to the inner main surface of the main portion along a leakage barrier joint extending in the length direction at least along the crotch portion, the article further comprising a pocket member extending along a pocket length in the length direction between a pocket rear edge and a pocket front edge, and in along a pocket width in the width direction between a pair of pocket side edges, wherein the pocket member is attached to the rear region of the main portion by a rear attachment element extending along the pocket rear edge, and by side attachment elements, each side attachment element extending along a pocket side edge such that the rear and side attachment elements form a closed faeces boundary between the pocket member and the main portion of the absorbent article with an opening at the pocket front edge towards the crotch region of the main portion,

the pocket member comprising a plurality of elastic members providing the pocket member with elastic characteristics along the width direction, and

wherein each one out of the side attachment elements are arranged outwardly of the respective leakage barrier joint as seen in the width direction.

This second alternative aspect may be combined with one or more of the features as set out in the above in relation to the first aspect, so as to obtain similar advantages.

The combination of the features of the two examples set out in the above, i.e. the combination of that each one out of the side attachment elements are arranged outwardly of the respective leakage barrier joint as seen in the width direction, and that the pocket member is arranged with no overlap in relation to the absorbent core as seen in the height direction, is believed to be particularly advantageous, since the two features together may contribute to the function of the pocket ensuring the opening thereof and at the same time contribute to a comfortable and relatively breathable article.

Thus, in a third alternative aspect not forming part of the invention as defined in the appended claims there is provided an absorbent article comprising a main portion extending in a length direction along a central length axis between a front edge and a rear edge of the absorbent article, and in a width direction along a central width axis, perpendicular to the central length axis between a pair of side edges of the absorbent article, and extending in a height direction perpendicular to the width and length directions, the main portion defining a main inner surface extending primarily along the width and length directions to be directed towards the wearer when the article is worn, and a main outer surface extending primarily along the width and length directions to be directed away from the wearer when the article is worn;

the main portion defining a front region comprising the front edge and a rear region comprising the rear edge, and a crotch region located between the front and rear regions, the front region and the rear region being adapted such that the front edge and the rear edge form part of a waist edge circumfering the waist of a user when the article is worn, and the main portion comprises an absorbent core extending at least in the crotch region of the main portion, wherein the absorbent article further comprises a pair of leakage barriers, each leakage barrier being joined to the inner main surface of the main portion along a leakage barrier joint extending in the length direction at least along the crotch portion, the article further comprising a pocket member extending along a pocket length in the length direction between a pocket rear edge and a pocket front edge, and in the width direction between a pair of pocket side edges,

wherein the pocket member is attached to the rear region of the main portion by a rear attachment element extending along the pocket rear edge and by side attachment elements, each side attachment element extending along a pocket side edge such that the rear and side attachment elements form a closed faeces boundary between the pocket member and the main portion of the absorbent article with an opening at the pocket front edge towards the crotch region of the main portion,

the pocket member comprising a plurality of elastic members providing the pocket member with elastic characteristics along the width direction, and

characterised in that each one out of the side attachment elements are arranged outwardly of the respective leakage barrier joint as seen in the width direction,

and the pocket member is arranged in a non-overlapping relationship with the absorbent core as seen in the height direction.

This third alternative aspect may also be combined with suitable features as described in the above in relation to the first aspect, so as to provide similar advantages.

For example, the third alternative aspect may be combined with features relating to the rear region of the main portion, or the entire main portion, being free from elastic members providing elasticity in the width direction. As such, the pocket member may form the desired rear elastic of the article.

For example, the sheet material of the pocket member, and/or the elastic elements may be as suggested in the above for the first aspect, to provide similar advantages.

For example, the more detailed features relating to the relationship between the pocket member and the absorbent core, and/or relating to the way in which the pocket member is attached to the main portion may be as suggested in the above for the first aspect, to provide similar advantages.

For example, the more detailed features relating to the relationship between the pocket member and the leakage barriers, and/or between the attachment regions and the leakage barriers may be as suggested in the above for the first aspect, to provide similar advantages.

In a second aspect, the object is achieved by an array of disposable articles. As such, there is provided an array of disposable articles comprising a plurality of articles according to the first aspect or alternatively, not forming part of the invention as defined in the appended claims, a plurality of articles according to one out of the first, second or third alternative aspects, wherein each article in the plurality of articles has an article length being different from the article length of the other articles in the array, and each article in the array of articles has a pocket length varying with less than 20% from the pocket length of the other articles in the array.

Optionally, each article in the array of articles has essentially the same pocket length.

As such, there may be provided an array of articles having different sizes, but using a pocket member having substantially the same pocket length for the different sizes. This may provide for efficient and cost-saving manufacture of the articles.

The array of disposable articles may comprise at least two articles having different article lengths. Optionally, the array of disposable articles may comprise a number of articles each having different article lengths, such as three, four or five articles.

As used herein, a direction projected area of an item is the area of a projection of the item onto a plane, wherein the normal of the plane is parallel to the direction.

With an "overlapping relationship" of a first item relative to a second item as seen in a direction is meant herein, at least a portion of the direction projected area of the first item is located within the direction projected area of the second item.

With a "completely overlapping relationship" a first item relative to a second item as seen in a direction is meant herein, that the direction projected area of the first item is located within the directed projected area of the second item. If a first item "completely overlaps" a second item, the direction projected area of the second item is located within the direction projected area of the first item.

With "non-overlapping" relationship of a first item relative to a second item as seen in a direction is meant herein, that that no portion of the direction projected area of the first item is located within the direction projected area of the second item.

With a "coinciding relationship" of a first item relative to a second item as seen in a direction is meant herein, that no portion of the direction projected area of the first item is located outside the direction projected area of the second item, and that no portion of the direction projected area of the second item is located outside the direction projected area of the first item.

With "inwards" in respect to a direction is meant herein towards the centre of the article along said direction.

With "outwards" in respect to a direction is meant herein away from the centre of the article along said direction.

With "extends along", i.e. where an item extends along an edge, includes configurations where the item coincides with the edge as well as configurations where item is arranged adjacent the edge, such as at a small distance therefrom.

Further options and advantages of the dispenser as disclosed herein are disclosed in the following description.

BRIEF DESCRIPTION OF THE DRAWINGS

Below follows a more detailed description of the absorbent articles as disclosed herein with reference to the appended drawings, wherein:

Fig. 1 is a view from the wearer-facing side of an example of an absorbent article in accordance with the first aspect, where the article is in an open state;
Fig. 2 is a similar view as in Fig. 1, but with the pocket member 20 removed such that the attachment elements attaching the pocket member to the main portion of the article are visible;
Fig. 3 is a top view of the pocket member of the article of Fig. 1 with attachment elements; and
Fig. 4 is a cross-sectional view through a plane extending along the length and height directions of the pocket member of the article of Fig. 1;
Fig. 5 is a cross-sectional view through a plane extending along the height and width directions of the article of Fig. 1 through one of the side attachment elements; and
Fig. 6 is a cross-sectional view through a plane extending along the height and length directions of the article of Fig. 1 through the rear attachment element.

Like reference numbers denote similar features throughout the figures. Reference numbers may be omitted in some figures for better visibility, in which case reference is made to the other figures.

DETAILED DESCRIPTION

Different aspects of the present disclosure will be described more fully hereinafter with reference to the enclosed drawings. The embodiments disclosed herein can, however, be realized in many different forms and should not be construed as being limited to the aspects set forth herein.

It is to be understood that the drawings are schematic and that individual components, such as layers of material are not necessarily drawn to scale.

Further, it should be understood that even if the description in the below is made with reference to a baby diaper, the article 1 need not necessarily be a baby diaper as illustrated but that the present disclosure and examples apply also e.g. to diapers intended for adult wearers.

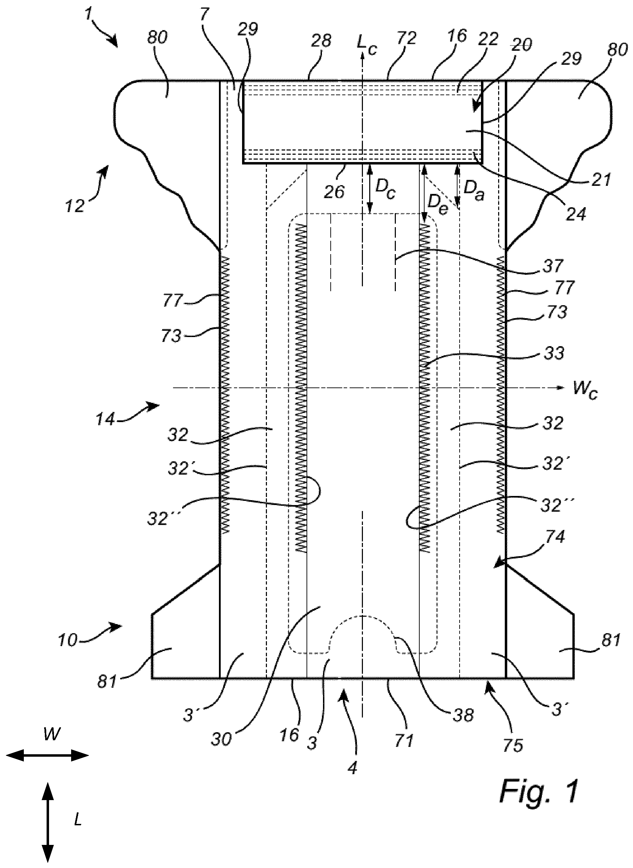

With initial reference to Fig. 1, there is shown an absorbent article 1 in the form of a baby diaper. The absorbent article 1 is shown in Fig. 1 in an unfolded and flat state with all elastic members in an extended state. The absorbent article 1 is seen from the surface which will be facing a wearer's body when the article is being worn.

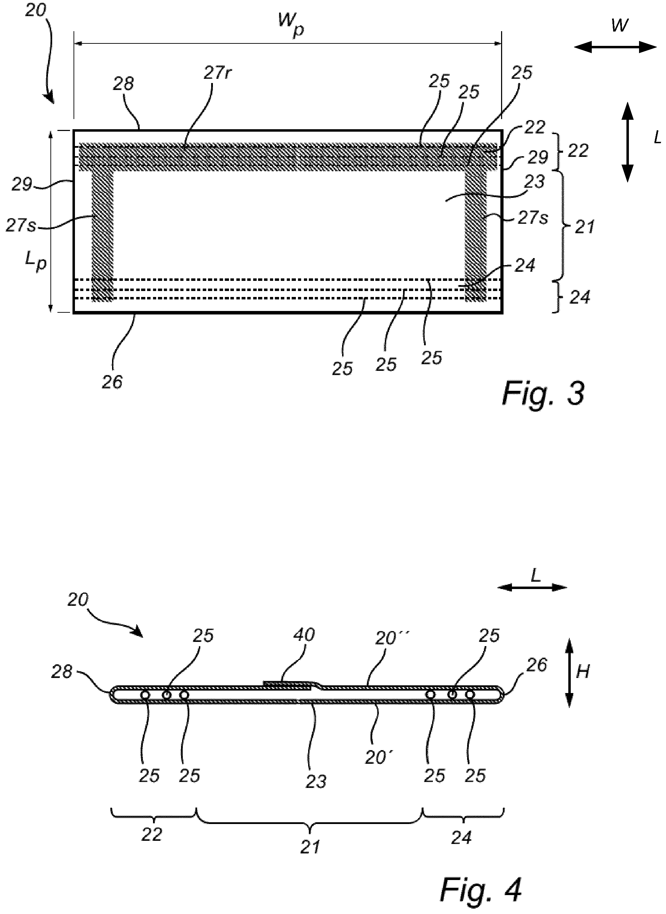

The absorbent article 1 comprises a main portion 7 extending in a length direction L along a central length axis Lc between a front edge 71 and a rear edge 72. The front edge 71 and the rear edge 72 of the main portion 7 also forms the front edge 71 and the rear edge 72 of the absorbent article 1. Further, the main portion 7 extends in a width direction W along a central width axis Wc between a pair of side edges 73. The pair of side edges 73 may, as illustrated in Fig. 1, also form the side edges 73 of the absorbent article. The absorbent article 1 also has a height direction H (see Figs 4 to 6) perpendicular to the length direction L and the width direction W.

The main portion 7 defines a front region 10 comprising the front edge 71, a rear region 12 comprising the rear edge 72, and a crotch region 14 located between the front and rear regions 10, 12.

The front region 10 and rear region 12 are adapted such that the front edge 71 and the rear edge 72 form part of a waist edge 16 of the article 1 circumfering the waist of a wearer when the article 1 is worn. In the absorbent article 1, the pair of side edges 73 of the main portion 7 may extend at least along said crotch region 14 so as to form side edges of the article 1 which are directed towards the legs of the wearer when the article 1 is worn. The side edges 73 may be provided with side elastics 77 providing elasticity to said side edges 73 along the length direction L.

The front region 10 is the part of the absorbent article which is intended to be oriented in a direction towards the belly of the wearer during use of the absorbent article 1 and the rear region 12 is the part of the absorbent article 1 which is intended to be oriented in a direction towards the buttocks of the wearer.

The absorbent article 1 may, as the article 1 in Fig. 1 be an open-type diaper having front side portions 81 attached to the main portion 7. The front side portions 81 extends from the side edges 73 in the front region 10 and adjacent the front edge 71 of the main portion 7. Further, rear side portions 80 may be extending from the side edges 73 in the rear region 12 and adjacent the rear edge 72 of the main portion 7. When putting on the absorbent article 1 on a wearer, the rear side portions 80 are brought forward towards the wearer's belly and are attached with fastener elements (not shown), such as hook-type fastener elements onto mating fastener elements such as loop-type fastener elements which are provided on the garment-facing side of the front region 10 of the diaper. As such, the article 1 is closed around the wearer such that the front edge 71 and the rear edge 72 form part of a continuous waist edge 16 around the wearer's body. Depending on the size and design of the article 1, the front and/or rear side portions 81, 80 may also form part of the continuous waist edge around the wearer's body.

It should also be noted that the fastening arrangement and the front and rear side portions are optional to the absorbent article 1, as the article may be designed for use as an absorbent insert, worn inside an outer shell. Such articles may be provided with releasable fastening adhesive arranged on the outer surface of the backsheet, to allow the article to be fastened inside the outer shell. The outer shell may be garment like.

The main portion 7 defines a main inner surface 74 extending primarily along said width and length directions W, L, and which will be directed towards the wearer when the article is worn. Further, the main portion 7 defines a main outer surface 75 extending primarily along said width and length direction W, L and which will be directed away from the wearer when the article 1 is worn.

As such, in a laid-out state as illustrated in Fig, 1 the main inner surface 74 is a generally planar surface extending between the front edge 71, the rear edge 72 and the side edges 73.

The main portion 7 further comprises an absorbent core 30. The absorbent core 30 may be sandwiched between a liquid permeable topsheet 3 and a liquid-impermeable backsheet 4. Furthermore, as illustrated in Fig. 1, the topsheet 3 and/or the backsheet 4 may extend outside of the absorbent core 30 as seen along the length direction L and/or the width direction W.

The absorbent core may comprise regions for shaping the absorbent core 30, such as less absorbent regions comprising less absorbent material than the adjacent regions of the absorbent core. For example, the absorbent core may comprise less absorbent regions in the form of longitudinal grooves 37 extending at least towards the rear end of the absorbent core 30. Alternatively or in addition, the absorbent core may comprise a cut-out, such as a cutout 38 as seen in from a front edge of the absorbent core. Such a cut-out may be adapted to provide a space which protects the sensitive part around the navel of a baby.

The topsheet 3 is arranged so as to form at least a part of the main inner surface 74 surface of the main portion 7, whereas the backsheet 4 (not shown) is arranged so as to form at least a part of the main outer surface 75 of the main portion 7.

For example, the backsheet 4 may extend continuously over the entire main portion 7, so as to form the main outer surface 75 of the entire main portion 7.

The topsheet 3 may in some variants also extend continuously over the entire main portion 7, so as to from the main inner surface 74 of the entire main portion 7.

However, and as in the illustrated example, the topsheet 3 may instead form a first part of the main inner surface 74, and one or more additional sheets may be arranged so as to form additional parts of the main inner surface 74. For example, and as in the illustrated example, parts of the main inner surface 74 may be formed by a leakage barrier sheet material 3', which forms part of the main inner surface 74 and which is used to form a leakage barrier 32, as will be further described in the below.

In the illustrated article, the topsheet 3 and the backsheet 4 extend outside the perimeter of the absorbent core 5 at the front, end and sides of the absorbent core 30. Further, the topsheet 3 and the backsheet 4 are joined outside the perimeter of the absorbent core 30.

It is also conceivable that the topsheet 3 and the backsheet 4 extend outside of the absorbent core 30 only along parts of the perimeter of the absorbent core 30, such as only along the side edges of the absorbent core 30 or only along one or both end edges of the absorbent core 30 or along the side edges and only one of the end edges.of the absorbent core 30.

The absorbent core 30 shown in Fig. 1 has a generally rectangular design. However, it is to be understood that the absorbent core may have any useful shape.

The topsheet 3, backsheet 4 and the absorbent core 30 may consist of any materials suitable for their purposes, as will be discussed in further detail below.

As disclosed herein, various types of materials may be used for the absorbent article 1.

The topsheet 3 which is arranged to face the wearer of the absorbent article 1 when the article is being worn may comprise or consist of a fluid permeable nonwoven fabric, film, mesh or foam. The topsheet may be made from thermoplastic material, such as thermoplastic synthetic fibers, film or netting. The topsheet 3 may be sufficiently liquid-permeable to allow discharged body fluids to penetrate through the thickness of the topsheet 3. Also, the topsheet 3 may suitably be manufactured from a material which is compliant and soft-feeling to the skin of the wearer. The topsheet 3 may consist of a single layer or may have a laminate structure comprising a plurality of layers, for example, two or more layers. The layers may be made of the same material, or some or all of the layers may be made of different materials.

The layer of the topsheet 3 or, for the case of a laminate structure, one, some, or all layers of the topsheet may be made of a single web of material or may have portions made of different materials, e.g., within different parts of the wearer-facing surface of the topsheet.

The layer of the topsheet 3 or, for the case of a laminate structure, one, some or all layers of the topsheet may be a nonwoven material, a perforated plastic film, a plastic or textile mesh, or a liquid permeable foam layer. The layer of the topsheet 3 or, for the case of a laminate structure, one, some or all of the layers of the topsheet may be, for example, a hydrophilic, non-apertured nonwoven web of fibers, such as natural fibers, e.g., cotton or pulp fibers, synthetic fibers, e.g., polyester or polypropylene fibers, or a combination of these fibers. The topsheet may have a basis weight in the range of 8-40 g/m$^2$. However, the disclosure is not limited to topsheets having this basis weight.

Furthermore, the topsheet may comprise at least one additive material such as a skin care composition.

Furthermore, the backsheet 4 may be constituted by a liquid-impermeable layer such as a polymeric film, for example a film of polyethylene or polypropylene. The backsheet 4 may be breathable. The materials which may be used for the backsheet 4 include thin and flexible fluid impermeable plastic films, or fluid impermeable nonwoven materials, fluid impermeable foams and fluid impermeable laminates. The backsheet 4 may be formed by a single layer, but may alternatively be formed by a multi-layered structure, i.e. a laminate, wherein at least one layer is fluid impermeable. Furthermore, the backsheet 4 may be elastic in any direction. Furthermore, the backsheet 4 may have a laminate structure comprising a liquid barrier sheet and a nonwoven layer arranged on top of each other (not shown in detail in the drawings), wherein the nonwoven layer is arranged at an outer side away from the wearer of the absorbent article 1 when worn.

The nonwoven layer may be made of thermoplastic polymer material fibers or filaments. The nonwoven layer may be formed by any of a variety of different processes, such as spunbonding, airlaying, meltblowing or bonded carded web formation processes. The nonwoven layer may be made of an SMS (spunbond/meltblown/spunbond) or SS (spunbond/ spunbond) nonwoven material of polypropylene or bicomponent fibers of polypropylene and polyethylene, or of a combination of such materials. The nonwoven layer may have a basis weight in the range of 5-40 g/m$^2$.

The liquid barrier sheet may be made of a plastic material, for example a thermoplastic film material, and/or a nonwoven material. For example, the liquid barrier sheet may be formed as a plastic layer, e.g., a thermoplastic layer, or a plastic film, e.g., a thermoplastic film. Forming the liquid barrier sheet of a plastic material, such as a thermoplastic film material, allows for a particularly good printability of the liquid barrier sheet. The liquid barrier sheet may also contain paper fibers. The liquid barrier sheet may be a liquid impermeable, breathable or non-breathable layer. The liquid barrier sheet may consist of a single layer or have a laminate structure with a plurality of layers, e.g., two or more layers, three or more layers, or four or more layers. The layers of the liquid barrier sheet may be laminated, bonded or attached to each other, for example, by thermo and/or mechanical bonding, such as thermo-sealing, ultrasonic bonding, such as ultrasonic welding, an adhesive or adhesives, stitching or the like. The liquid barrier sheet may be a breathable microporous film. The microporous film may be made of a material comprising at least two basic components, namely a thermoplastic elastomeric polyolefin polymer and a filler. These components and, in some embodiments, additional other components may be mixed together, heated and subsequently extruded into a mono-layer or multi-layer film using any one of various film-producing processes, such as cast embossed, chill and flat cast, and blown film processes.

The absorbent core 30 may include an absorbent component and may include further absorbent components such as components which provide liquid acquisition and liquid distribution. The absorbent core 30 is disposed between the topsheet 3 and the backsheet 4 to absorb the liquid, such as urine or other bodily fluids, which has passed through the topsheet 3. The absorbent component may be a single-layer structure or may be a layered structure, e.g. within the coherent area. The absorbent component may comprise suitable amounts of superabsorbent material. Such superabsorbent material is well known in the field of absorbent articles, and is constituted by a water-swellable and water-insoluble material which is capable of absorbing large quantities of fluid upon formation of a hydrogel. The absorbent component may contain superabsorbent material in the form of fibers or particles of absorbent polymer material. For example, the superabsorbent material may be surface cross-linked, partially neutralized polyacrylates. The superabsorbent material, e.g., the superabsorbent fibers or particles, may be mixed with other absorbent or liquid uptake material or materials, such as cellulose fluff pulp, and/or arranged in pockets or layers in the absorbent component. The amount of superabsorbent material and pulp in the absorbent component may be within the range from 0 to 50 weight % pulp fibers and within the range from 50 to100 weight % superabsorbent material, or within the range from 0 to 30 weight % pulp fibers and within the range from 70 to100 weight % superabsorbent material.

The absorbent component may further comprise components for improving properties of the absorbent core 30, such as core integrity and strength. For example, the absorbent component may comprise a binder or binders, such as binder fibers. Resilient fibers, chemically stiffened fibers, etc. may be present in the absorbent component to counteract wet-collapse of cellulosic fibers. Such fibers may also be useful in retaining a fluid transporting capillary network in the absorbent component so that absorbent fluid may be distributed in the absorbent component and be absorbed by superabsorbent material also in parts of the absorbent component outside the initial wetting area of the absorbent article.

Furthermore, the various layers and components of the absorbent article 1 may be attached by means of adhesive material, as known in the art. Such adhesive is not shown in the drawings.

One or more additional layers may be provided in the absorbent article 1. For example, an acquisition layer may be arranged between the absorbent core 30 and the topsheet 3. Such an additional layer may for example be in the form of an airlaid layer, a spunlace layer, a high-loft fiber material, an open-cell or perforated foam or any other type of material layer or combination of layers which may be used in an absorbent article to act as a liquid handling layer providing functions such as liquid acquisition, liquid absorption and liquid distribution. A liquid acquisition layer is adapted to quickly receive and temporarily store discharged liquid before the liquid can be absorbed by the absorbent component. Such acquisition layer may be composed of for example airlaid nonwoven, spunlace nonwoven, high loft nonwoven or foam materials. An airlaid nonwoven may be produced wood pulp fluff fibers which are dispersed and suspended in a fast-moving air stream and condensed onto a moving screen by means of pressure and vacuum.

The absorbent article 1 may, as the example in Fig. 1 also have leakage barriers 32 arranged to the main portion 7 of the absorbent article 1. The leakage barriers 32 may extend generally along the length direction L and on each side of the absorbent core 30. The leakage barriers 32 may have an extension at least in the crotch region 14 of the article 1. However, the leakage barriers 32 may also extend not only in the crotch region 14 but also, as in the illustrated variant, in a part of the front region 10 and/or the rear region 12. For example, the leakage barriers 32 may extend substantially over the entire length of the main portion 7.

Each leakage barrier 32 is joined to the main portion 7 along a leakage barrier joint 32' extending at least in said length direction L. The leakage barrier 32 extends from the leakage barrier joint 32' along a leakage barrier width extending in a plane perpendicular to the length direction L to a leakage barrier edge 32".

The leakage barrier 32 may be formed by a strip of material being attached by the leakage barrier joint 32' e.g. to a topsheet 3 of the article. However, the leakage barrier 32 may also be formed e.g. by a fold of topsheet material 3 extending from the leakage barrier joint 32'.

For example, the leakage barrier 32 may be formed by a first topsheet material 3 extending on the inside of the leakage barrier joint 32 towards the length axis L, and by a leakage barrier sheet material 3' extending on the outside of the leakage barrier joint 32'. Thus a portion of the first topsheet material 3 and a portion of the second topsheet material 3' may both extend from the leakage barrier joint 32' at the inner main surface 74 of the main portion 7 in the height direction H, and be joined so as to form the leakage barrier 32.

As such, the leakage barrier 32 may display a leakage barrier active region being provided at least in the crotch portion of the article, wherein the leakage barrier 32 extends freely from the leakage barrier joint 32' to a free leakage barrier edge 32". Thus, in the leakage barrier active region, the full leakage barrier width is available such that the leakage barrier 32 may rise to form a barrier towards the wearer's legs. The free leakage barrier edge 32" may be provided with a leakage barrier elastic member 33 to provide elasticity along the length direction L of the free leakage barrier edge 32".

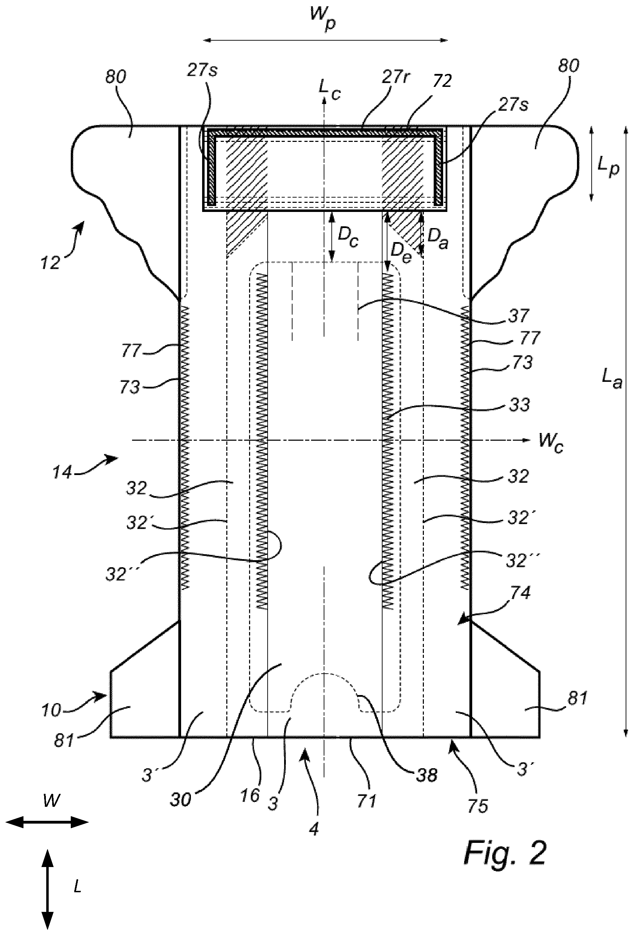

Towards the front region 10 and rear region 12 of the article 1, the leakage barriers 32 may display a leakage barrier fastening region (striped in Fig. 2), wherein the leakage barrier 32 is fastened to the inner main surface 74 of the main portion 7, i.e. to the topsheet 3. In the leakage barrier fastening region, the leakage barrier 32 may be fastened to the inner main surface 74 of the main portion 7 at a location between the leakage barrier joint 32' and the leakage barrier edge 32', such that the leakage barrier 32 may only extend freely in a plane perpendicular to the length direction L along a leakage barrier unfastened width being less than the original leakage barrier width. As such, in the leakage barrier fastening region, the leakage barrier 32 is directed towards the inner main surface 74 of the main portion 7 rather than raised to form a barrier towards the wearer's legs. For example, in the leakage barrier fastening region, the leakage barrier unfastened width may be less than 20% of the leakage barrier width. Or, the leakage barrier unfastened width may be 0, indicating that the leakage barrier edge 32" is fastened to the main portion 1.

As exemplified in Fig. 1, the rear region 12 comprises a pocket member 20 arranged to form a pocket being open towards the crotch region 14 of the absorbent article 1. To this end, the pocket member 20 is arranged on the main inner surface 74 of the main portion 7.

The pocket member 20 extends along a pocket length Lp in the length direction L between a pocket rear edge 28 and a pocket front edge 26. Also, the pocket member 20 extends along a pocket width Wp in the width direction, between a pair of pocket side edges 29.

The pocket member 20 may, as in the illustrated variant, be generally rectangular, which is advantageous in view of manufacture and function. However, other options are feasible. The pocket length Lp and pocket width Wp refer in cases with irregular length and width to the maximum pocket length and pocket width as seen when the pocket 20 is arranged on the article 1.

As seen in Figs 1, 3 and 4, the pocket member 20 comprises a plurality of elastic members 25 providing the pocket member 20 with elastic characteristics along the width direction W.

The elastic members 25 are distributed over the pocket length Lp of the pocket member 20 so as to form a first elastic zone 22 extending from the pocket rear edge 28 towards the front of the article 1 along the length direction L and a second elastic zone 24 extending from the pocket front edge 26 and towards the rear of the article 1 along the length direction L.

The elastic members 25 display a first average distribution as seen along the length direction L in the first elastic zone 22. The elastic members 25 display a second average distribution as seen along the length direction L in the second elastic zone 24. As illustrated in the Figs, the first and second average distribution may be the same.

Further, an intermediate zone 21 extends in the length direction L from the first elastic zone 22 to the second elastic zone 24. The intermediate zone is defined between a frontmost elastic member 25 of the second elastic zone 24 and a rearmost elastic member of the first elastic zone 22. The elastic members 25 display an average distribution in the intermediate zone which is less than 0.5 elastic members per cm as seen in the length direction L. Accordingly, the intermediate zone will be relatively inelastic, such that the portion of the pocket member 20 forming the intermediate zone is essentially unbiased along the width direction W, allowing the pocket member 20 to form a volume between the pocket member 20 and the remainder of the absorbent article 1, e.g. the main inner surface 74 of the main portion 7 for containing faeces.

As in the illustrated example, the intermediate zone 21 may be free from elastic members 25.

The intermediate zone 21 extends along at least 20% of a pocket length Lp (see Fig. 2) as defined from the pocket front edge 26 to the pocket rear edge 28 along the length direction L. Thus, the intermediate zone 21 of the pocket member 20 allows for the pocket formed by the pocket member 20 to provide a desired volume for taking up faeces.

The first and second average distribution of the elastic members 25 in each of the first and second elastic zone 22, 24 is greater than in the intermediate zone 21, which implies that in the first and second elastic zone 22, 24, the pocket member 20 is biased towards a contracted condition as seen in the width direction W.

As such, the elastic members 25 of the first elastic zone 22 contracting the rear region 12 adjacent the rear waist edge 16 of the absorbent article 1, contributes to the rear region 12 adjacent the rear waist edge 16 being biased towards the back of the wearer so as to comfortably conform to the wearer's movements. Thus, the elastic members 25 of the first elastic zone 22 contribute to the general comfort and fit of the article 1.

As in the variant illustrated in the Figures, the rear region 12 of the main portion 7 may be free from other elastic members but the elastic members 25 of the first elastic zone 22 over a width extension corresponding to a width of the first elastic zone 22, optionally also corresponding to a width of the pocket Wp. In other words, the elastic members 25 of the first elastic zone 22 may be the only elastic members in the rear region 12 which provide elasticity along the width direction W of the rear region 12 the main portion 7.

Further, as in illustrated variant, the front region 10 of the main portion 7 may be free from elastic members providing elasticity along the width direction W of the front region 10.

Thus, the elastic members 25 of the first zone 22 of the pocket member 20 may provide sufficient waist elasticity to the main portion 7, such that additional waist elastic members may not be necessary.

The elastic members 25 of the second elastic zone 24 are located adjacent the front edge 26 of the pocket member 20, which front edge 26 will form the opening of the pocket towards the crotch region 14 of the article 1. By the elastic members 25 of the second elastic zone 24 contracting the second elastic zone 24, the second elastic zone 24 will be urged towards the back of the user when the article 1 is worn, thus ensuring that the pocket opening is expanding so as to allow faeces to enter the volume formed between the pocket member 20 and the inner main surface 74 of the main portion 7 e.g. the top sheet 3. Thus, the elastic members 25 of the second elastic zone 24 ensures that the pocket formed by the pocket member 20 will be efficient for gathering faeces.

In view of the above, the pocket member 20 of the absorbent articles 1 disclosed herein, provides a well-functioning faeces pocket which may be easily assembled.

Further, as will be more described in the below, the intermediate zone 21 of the pocket member 20 allows for a pocket being advantageous in terms of breathability, having no or relatively scarcely distributed elastic members.

The intermediate zone 21 may extend over no more than 80 % of the pocket length Lp as seen in the length direction L.

For example, the intermediate zone 21 may extend from 20 to 50% of the pocket length Lp, such as from 20 to 40% of the pocket length.

As in the illustrated example, each one out of the first and second elastic zones 22, 24 may extend over a length being less than the length of the intermediate zone 21 as seen in the length direction L. A such, the intermediate zone 21 has the greatest length of the three zones (first elastic, second elastic, and intermediate) of the pocket member 20, to the benefit of e.g. volume and breathability.

As such, the elastic members 25 are arranged so as to provide the desired contraction of the pocket member 20 adjacent the pocket front edge 26 and the pocket rear edge 28 respectively thereof, while the intermediate 21 may provide for sufficient volume of the pocket formed.

For example, each one out of the first and second elastic zones 22, 24 may extend over at least 10% of the pocket length Lp as seen in the length direction L. For example, each one out of the first and second elastic zones may extend over no more than 40% of the pocket length Lp, such as over no more than 30% of the pocket length Lp.

As illustrated in the Figures, the elastic members 25 may extend substantially in parallel to the width direction W.

As also illustrated in the Figures, the pocket rear edge 28 of the pocket member 20 may extend substantially in parallel to the width direction W. The elastic members 25 may extend substantially in parallel to the pocket rearedge 28 of the pocket member 20.

Further, the pocket front edge 26 of the pocket member 20 may extend substantially in parallel to the width direction W. The elastic members 25 may extend substantially in parallel to the pocket front edge 26 of the pocket member 20.

The elastic members 25 are arranged to provide elasticity in a direction parallel to the width direction W of the article. However, the elastic members 25 provide no elasticity in a direction perpendicular to the width direction W of the article 1, i.e. along the length direction L of the article 1.

With reference to the pocket member 20, the elastic members 25 are arranged to provide elasticity in a direction parallel to a pocket width Wp, but not in a direction parallel to a pocket length Lp.

The elastic members 25 may extend continuously over the pocket width Wp.

The average distribution of elastic members in the first and/or second elastic zone 22, 24 may be selected so as to achieve the desired contraction of the pocket member 20, without unnecessarily adding extra material to the pocket member 20.

In some variants, the first elastic zone 22 may display an average distribution of elastic members 25 which is different from the average distribution of elastic members 25 of the second elastic zone.

However, in other variants, the first and the second elastic zone 22, 24 may display the same average distribution of elastic members 25.

The first and/or second elastic zone 22, 24 may for example display an average distribution of elastic members being greater than 0.5 members /cm, as seen along the length direction L. For example, the average distribution may be greater than or equal to 1 member /cm. For example, the average distribution may be no more than 3 members/cm. For example, the average distribution may be from 1 to 3 members/cm. For example, the average distribution may be 2 members/cm,

The first and the second elastic zone 22, 24 each comprises at least one elastic member 25.

The first and/or the second elastic zone 22, 24 may each comprise a plurality of elastic members 25, wherein each elastic member 25 is spaced from neighbouring elastic members 25 as seen in the length direction L.

The distance between the elastic members has an impact on the contractive force provided by the elastics, and hence on the pressure provided by the elastics towards the wearers back at the waist region of the article, and the urging of the front edge of the pocket member towards the back of the wearer to open up the pocket.

Generally, an increased distance between the threads reduces the pressure and a reduced distance between the threads increases the pressure provided.

As such, a distance as seen in the length direction L between two neighbouring elastic members 25 may be at least 3 mm, such as from 3 to 7 mm, as seen in the length direction L. For example, a distance as seen in the length direction between two neighbouring elastic members 25 may be 5 mm.

For example, in the first and/or second elastic zone 22,24, each elastic member 25 may spaced from neighbouring elastic members 25 by the same distance as seen in the length direction L. In other words, the elastic members 25 may be regularly distributed along the length direction L in the first and/or second elastic zone 22, 24.

The first and/or second elastic zone 22, 24 may each comprise no more than seven elastic members 25, such as no more than five elastic members. In some variants, as in the illustrated article the first and/or second elastic zone may comprise three elastic members 25.

The elastic members 25 may, as in the illustrated variant, be elastic threads.

Thus, the elastic threads may be arranged so as to extend in parallel to each other and generally in parallel to the width direction W of the article 1. For example, the elastic threads may extend along substantially the entire width Wp of the pocket member 20.

Elastic threads may provide elasticity while having a relatively low thickness, hence having a relatively low impact on the breathability of the pocket member 20.

For example, the elastic threads may be elastic threads with from 400 to 1100 dtex.

The elasticity of the elastic members 25 in the elastic zones 22, 24 may be selected in view of the desired properties as mentioned in the above, and adjusted together with the number of elastic members and distribution thereof to arrive at a desired result.

For example, one or more, preferably all of the elastic members 25 in the first and second elastic zones 22,24 may have an elasticity of 100-250%.

The pocket member may comprise a sheet material 23.

In some variants, the pocket member may comprise one layer of sheet material 23 to which the elastic members 25 is joined, e.g. by gluing or welding.

In other variants, the pocket member 20 may comprise two layers of sheet material 23. In this case, the elastic members 25 may be positioned between the two layers of sheet material 23.

For example, and as illustrated in Fig. 4, the two layers may be formed by a continuous piece of sheet material 23 being folded around the elastic members 25, and joined by a joint 40 to form the pocket member.

The sheet material 23 may be folded such that the joint 40 is located along the intermediate zone 21 of the pocket member 20. Thus, the joint 40nmay be formed so as to extend generally in parallel to the width Wp of the pocket member 20. The pocket front edge 26 of the pocket member 20 and the pocket rear edge 28 of the pocket member 20 are thus folded edges of sheet material 23.

With the joint 40 located in the intermediate zone 21 of the pocket member 20, the joint 40 may be spaced from the first and second elastic regions 22, 24, and thus any impact of the joint on the elasticity of the elastic region(s) may be avoided.

The joint 40 may be arranged so as to have a length as seen along the length direction L being at least 2mm, such as from 2 to 4 mm.

The joint 40 may have a width as seen in the width direction W corresponding to the width Lw of the pocket.

As such, the intermediate zone 21 may consist of the sheet material 23 only or, if any joint 40 is present, of the sheet material 23 comprising the joint 40.

For example, out of a total planar area of the intermediate zone 21, at least 90 % may comprise the sheet material only.

For example, out of a total planar area of the pocket member 20, at least 75% comprises sheet material 23 only.

The "planar area" as used in the above is the area as measured in a planar condition with any elasticity from any elastic members removed.

For example, the sheet material 23 has a basis weight from 10 gsm to 60 gsm, such as from 20 gsm to 30 gsm.

The sheet material may be a nonwoven having a basis weight from 10 gsm to 30 gsm.

As set out in the above, the sheet material may have an air permeability and/or a Material Water Vapor Transmission rate suitable for the material to be perceived as breathable and thus comfortable to the wearer.

For example, the sheet material may be a spunbond nonwoven comprising PP fibers, having a basis weight of 23 gsm.

As mentioned in the above, the absorbent article 1 comprises an absorbent core 30. As illustrated in Figs 1 and 2, the pocket member 20 may be arranged in a non-overlapping relationship with the absorbent core 30, as seen in the height direction H. Thus, a distance between the pocket front edge 26 of the pocket member 20 and a rearmost edge of the core 30 is provided.

By avoiding overlap between the pocket member 20 and the absorbent core 30, it is avoided to form a region of the article 1 where breathability may be adversely impacted by the presence of several material layers over one another in the height direction H of the article 1.

Further, by providing a distance between the absorbent core 30 and the pocket member 2, it is avoided that the absorbent core 30 adversely impacts the function of the pocket member 20. For example, it is avoided that the absorbent core 30 blocks the opening of the pocket formed by the pocket member 20 as the absorbent core 30 expands after wetting thereof.

For example, the distance in the length direction L between the pocket front edge 26 and the absorbent core 30 may be 5 mm or more.

As mentioned in the above, and as illustrated in Fig. 2, the pocket member 20 is attached to the rear region 12 of the main portion 7, for example to the main inner surface 74 of the main portion 7. As illustrated in Fig. 2, the attachment is be made via attachment elements 27r, 27s forming a closed faeces boundary between the pocket member 20 and the main portion 7 apart from at the opening towards the crotch region 14 of the absorbent article 1.

As mentioned in the above, the pocket member 20 extends between a first side edge and a second pocket side edge 29 in the width direction W, and between the pocket front edge 26 and the pocket rear edge 28 in the length direction L. The rear attachment element 27r extends along the pocket rear edge 28 and the side attachment elements 27s along the first and second pocket side edges 29, respectively

As in the illustrated variant, the rear attachment element 27r may extend along substantially the entire pocket rear edge 28, and the side attachment elements 27s along substantially the entire first and second side edges 29 of the pocket member 20.

The attachment may be made using any suitable attachment method such as welding or gluing. For example, the attachment elements 27r, 27s may comprise an adhesive.

To form a closed faeces boundary between the pocket member 20 and the inner main surface 74 apart from at the opening towards the crotch region 14 of the main portion 7, the rear and side attachment elements 27r, 27s may for example be continuous attachment elements.

Notably, as illustrated in Fig. 1, the pocket member 20 is shown with the elastic members 25 in a fully extended condition. Hence the sheet material 23 of the pocket member 20 may as illustrated be attached to the underlaying main portion 7 with the sheet material 23 in a substantially flat state. In other words, the sheet material 23 has no creases or gathers.

Thus, the attachment of the pocket member 20 to the rear region 12 of the main portion 7 may be made such that, when the article 1 is in an open laid-out state, the area formed between the attachment elements 27 on the inner main surface 74 of the main portion 7, essentially corresponds to the area of the sheet material 23 of the pocket member 20 between the attachment elements 27.

This implies that, when the first and second elastic regions 22, 24 of the pocket member 20 are released, the first elastic region 22 may contract the rear region 12 adjacent the rear waist edge 16', working as a waist elastic, and the second elastic region 24 may contract the pocket member 20 adjacent the pocket front edge 26. At the same time, the portion of the remainder of the article 1 which extends in the width direction W between the attachment elements 27 extending along the side edges 29 of the pocket member 20, will be urged towards each other at least at the location of the second elastic region 24. In use, this will have the effect that the portion of the main portion 7 extending between the side edges 19 of the pocket member bulging away from the wearer, whereas at least a front portion of the pocket member 20 is contracted and moved towards the wearer, hence resulting in the desired opening of the pocket.

Further, as illustrated in Figs 4 to 6, the pocket member 20 forms a first surface 20' and a second surface 20" opposite to said first surface 20'. The pocket member 20 is arranged with said first surface 20' forming a wearer facing surface, facing towards the wearer when the article 1 is worn. The second surface 20" is forming a main portion facing surface, facing the main portion 7. The attachment elements 27r, 27s are arranged to attach the second surface 20" to the main inner surface 74 of said main portion 7. This implies that the attachment of the pocket member 20 to the main portion 7 is made with the pocket member 20 in a flat, unfolded state.

The area of the rear and side attachment elements 27r, 27s may be selected so as to provide satisfactory fastening of the pocket member 20 to the main portion 7. For example, the rear and side attachment elements 27r, 27s may be arranged so as to cover 60% or less of a total planar surface area A of the pocket member 20, for example 50% or less of the total planar surface area. This allows for the unfastened area of the pocket member 20 to be active in forming a volume for the faeces. Further, using lesser amounts of adhesive has a positive impact on the breathability of the article 1 as a whole.

As illustrated in Fig. 2, the rear attachment element 27r may be arranged in an at least partly overlapping relationship with the first elastic region 22, such as in a coinciding relationship with the first elastic region 22, as seen along the height direction H. As such, the elasticity provided by the first elastic region 22 may act so as to elasticise also the main portion 7 underlaying the first elastic region 22, and thus the first elastic region 22 is efficient as a waist elastic of the article 1.

With the first elastic region 22 serving as a waist elastic, at least the rear portion 12 of the main portion 7 may be free from elastics providing elasticity along the width direction W.

For example, as in the illustrated variant, the entire main portion 7 may be free from elastics providing elasticity along the width direction W.

As illustrated in the Figs, the pocket member 20 may be arranged in a non-overlapping relationship with the active region of the leakage barriers 32. As such, the pocket member 20 may be arranged in a non-overlapping relationship with the leakage barrier elastic members 32''', as seen along the height direction of the article.

The pocket member 20 may, as exemplified in the Figs., be arranged in an overlapping relationship with the leakage barrier fastening region (dashed in Fig. 2). Thus, the pocket member 20 may extend in the width direction W over the pair of leakage barrier joints 32'. Accordingly, the side barriers formed by the leakage barriers 32 may work together with the pocket formed by the pocket member 20 for gathering faeces material, although the function of the pocket member 20 is not dependent e.g. on the raising of the leakage barriers 32.

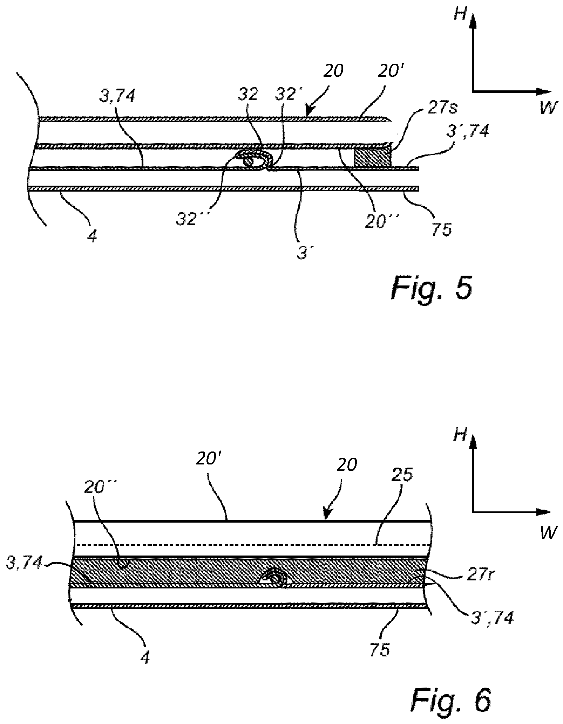

Rather, and as illustrated in Figs. 5 and 6, the rear and side attachment elements 27s, 27r provides for direct attachment of the pocket member 20 to the main inner surface 74 of the main portion 7. As outlined in the above, in the rear region 12, the main portion 7 may comprise relatively few layers of material. For example, as illustrated in the Figs., the attachment elements 27r, 27s may be arranged in an overlapping relationship only with portions of the rear region 12 which comprises no more than two sheet materials, i.e. a main inner surface forming material and a main outer surface forming material, with the exception of a minor portion of the leakage barrier fastening region which may form a partially overlapping relationship with the rear attachment element 27r. As such, the attachment elements 27r, 27s are arranged in an overlapping relationship only with portions of the main portion 7 which comprises no more than two sheet materials, i.e. the main inner surface forming material and the main outer surface forming material.

In the illustrated example, as seen in Fig. 5, the main inner surface 74 is formed by a leakage barrier sheet material 3' at the location of the side attachment element 27s. The main outer surface 75 is formed by the backsheet material 4.

Further, as seen in Fig. 6, the main inner surface 74 is formed by a topsheet material 3 at an inward location of the rear attachment element 27r as shown in Fig. 6, and by a leakage barrier sheet material 3' at outward portions of the rear attachment element 27r as seen in Fig. 2. The main outer surface 75 is formed by a backsheet material 4 at the location of the rear attachment element 27r.

It is to be noted that Figs 5 and 6 are not drawn to scale and that the proportions of e.g. the leakage barrier 32 may vary. Further, layers such as the two layers in the pocket member 20, and sheets such as the top sheet 3 and the back sheet 4, which are joined in the article 1 are shown as separated for better visibility.

As such, the article 1 may also in the areas of the attachment elements 27s, 27r be relatively thin, comprising relatively few layers, to the advantage of comfort and breathability of the article 1.

As illustrated in the Figs, the side attachment elements 27s may be arranged outside of the leakage barrier joint 32' as seen in the width direction W of the article. Thus, the side attachment elements 27s may be provide for direct attachment of the pocket member 20 to the main inner surface 74 of the main portion 7, while providing for the pocket member 20 extending in the width direction W over the pair of leakage barrier joints 32'.

As mentioned in the above, the absorbent article 1 may comprise elastic side portions 81 extending along the width direction W on each side of the rear region 12 of the main portion 7. The pocket member 20 is arranged in a non-overlapping relationship with the elastic side portions 81 as seen in the height direction H. Again, this provides for the pocket member being attached direction to the inner main surface 74 of the main portion 7.

The article 1 has an article length La along the length direction L, wherein a pocket length proportion, being the pocket length Lp/ the article length La, is from 5% to 20 %. The pocket total length Lp may for example be from 20 to 60 mm.

The absorbent article 1 may, as the article illustrated in the Figs, be a disposable absorbent article.

In a second aspect, there is provided an array of disposable articles comprising a plurality of articles 1 as described herein, wherein each article 1 in the plurality of articles has an article length La being different from the article length La of the other articles 1 in the array, and each article 1 in the array of articles has a pocket length Lp varying with less than 20% from the pocket length Lp of the other articles 1, 7 in the array. As such, each article 1 in the array of articles may have essentially the same pocket length Lp. Such an array of articles 1 may provide for efficient manufacture of the article.

When manufacturing an article 1, a pocket member material strip may be provided having a width corresponding to the pocket length Lp, and from which pocket member material strip pocket members having a desired pocket width Wp may be cut. Thus, for forming an array of articles 1 wherein each article in the array has the same pocket length Lp, the same pocket member material strip may be used for all articles in the array.

The array of articles may comprise at least two articles having different article lengths La, such as two articles of different sizes, e.g. intended for babies of different age.

Numerous variants and options of the articles disclosed here will be conceivable by the person skilled in the art within the scope of protection defined by the appended claims.

**Claims**

1. An absorbent article (1) comprising a main portion (7) extending in a length direction (L) along a central length axis (Lc) between a front edge (71) and a rear edge (72) of said absorbent article (1), and in a width direction (W) along a central width axis (Wc), perpendicular to said central length axis (L) between a pair of side edges (73) of said absorbent article (1), and extending in a height direction (H) perpendicular to said width and length directions (L, W),

   the main portion (7) defining a main inner surface (74) extending primarily along said width and length directions (W, L) to be directed towards the wearer when the article is worn, and a main outer surface (75) extending primarily along said width and length directions (W, L) to be directed away from the wearer when the article is worn;
   the main portion defining
   a front region (10) comprising said front edge (71) and a rear region (72) comprising said rear edge (72), and a crotch region (14) located between the front and rear regions (10, 12), the front region (10) and the rear region (12) being adapted such that said front edge (71) and said rear edge (72) form part of a waist edge (16) circumfering the waist of a user when the article (1) is worn,
   the article (1) further comprising a pocket member (20) extending along a pocket length (Lp) in said length direction (L) between a pocket rear edge (28) and a pocket front edge (26), and along a pocket width (Wp) in said width direction (W) between a pair of pocket side edges (29), wherein said pocket member (20) is attached to said rear region (12) of the main portion (7) by a rear attachment element (27r) extending along the pocket rear edge (28) and by side attachment elements (27s), each side attachment element (27s) extending along a pocket side edge (29) such that said rear and side attachment elements (27r, 27s) form a closed faeces boundary between said pocket member (20) and said main portion (7) of said absorbent article (1) with an opening at said pocket front edge (26) towards the crotch region (14) of said main portion (7),
   **characterised by**
   said pocket member (20) comprising a plurality of elastic members (25) providing said pocket member (20) with elastic characteristics along said width direction (W),
   said elastic members (25) being distributed over the pocket length (Lp) of said pocket member (20) so as to form
   a first elastic zone (22) extending from said pocket rear edge (28) along the length direction (L) and
   a second elastic zone (24) extending from said pocket front edge (26) along the length direction (L), and an intermediate zone (21) extending in said length direction (L) from said first elastic zone (22) to said second elastic zone (24) and over at least 20% of the pocket length (Lp), and an average distribution of said elastic members in said intermediate zone is less than 0.5 elastic members per cm as seen in the length direction (L), and an average distribution of said elastic members (25) in each of said first and second elastic zone (22, 24) is greater than said average distribution of elastic members in said intermediate zone (21), as seen in the length direction (L).

2. The disposable article according to claim 1, wherein said intermediate zone (21) extends in said length direction (L) over 80% or less of the pocket length (Lp).

3. The disposable article according to claim 1 or 2, wherein each one out of the first and second elastic zones (22, 24) extends over at least 10% of the pocket length (Lp) as seen in the length direction (L).

4. The absorbent article according to any one of the preceding claims, wherein, in each of said first and/or second elastic zone (22, 24), an average distribution of said elastic members (25) is greater than 0.5 members/cm, such as greater than 1 member/cm, such as from 1 to 3 members/cm as seen in the length direction (L).

5. The absorbent article according to any one of the preceding claims, wherein, in each first and/or second elastic zone, a distance as seen in the length direction L between each pair of two neighbouring elastic members (25) is at least 3 mm, such as within the range from 3 to 7 mm, as seen in the length direction (L).

6. The absorbent article according to any one of the preceding claims, wherein said elastic members (25) are elastic threads.

7. The absorbent article according to any one of the preceding claims, wherein one or more, preferably all of the elastic members (25) in said first and second elastic zones (22,24) have an elasticity within the range of from 100 % to 250%.

8. The absorbent article according to any one of the preceding claims, wherein said first elastic zone (22) is arranged adjacent said rear edge (72) of the main portion (7), so as to form a rear waist elastic, contributing to the elasticity of the rear region (12) of said main portion (7) around the waist of the user when said article (1) is worn.

9. The absorbent article according to any one of the preceding claims, wherein said rear region (12) of said main portion (7) is free from elastic members providing elasticity in the width direction (W).

10. The absorbent article according to any one of the preceding claims, wherein said main portion (7) is free from elastic members providing elasticity in the width direction (W).

11. The absorbent article according to any one of the preceding claims, wherein said rear attachment element (27r) at least partly, preferably completely, overlaps the first elastic zone (22) as seen along the height direction (H).

12. The absorbent article according to any one of the previous claims, wherein said pocket member (20) comprises a sheet material (23), such as a nonwoven material,

   wherein said pocket member (20) comprises two layers of sheet material (23),
   wherein said elastic members (25) are positioned between said two layers of sheet material (23).

13. The absorbent article according to claim 12, wherein said two layers are formed by a continuous piece of sheet material (23) being folded around said elastic members (25), and joined by a joint (40) to form said pocket member (20).

14. The absorbent article according to any one of the claims 12 to 13, wherein said sheet material (23) has a basis weight within the range from 10 gsm to 60 gsm, such as said sheet material being a nonwoven material having a basis weight within the range from 10 gsm to 30 gsm.

15. The absorbent article according to any one of the claims 12 to 14, wherein said sheet material (23) has an air permeability of at least 1000 l/m2/s, such as at least 2000 l/m2/s and/or a Material Water Vapor Transmission rate of at least 2000 g/m2/24h, such as at least 4000 g/m2/24 h.

16. The absorbent article according to any one of the claims 12 to 15, wherein said sheet material (23) is a nonwoven material comprising fibres composed out of one or more components, the components being polymer components such as PP or PE, such as PP-PE fibers and/or PP-PP fibers.

17. The absorbent article according to any one of the previous claims wherein said main portion (7) comprises an absorbent core (30) extending at least in the crotch region (14) of the main portion (7),

   wherein said main portion (7) comprises a top sheet (3) forming at least a part of said inner main surface (74), and a back sheet (4) forming at least a part of said outer main surface (75), said absorbent core (30) being arranged between said top sheet (3) and said back sheet (4), wherein said pocket member (20) is arranged in a non-overlapping relationship in relation to said absorbent core (30) as seen in the height direction (H).

18. The absorbent article according to claim 17, wherein a distance (Dc) in the length direction (L) between said pocket front edge (26) and said absorbent core (30) is 5 mm or more.

19. The absorbent article according to any one of the claims 17 to 18, wherein each one of said side attachment elements (27s) is arranged outward of said absorbent core (30), as seen along the width direction (W).

20. The absorbent article according to any one of the previous claims, wherein said absorbent article (1) further comprises a pair of leakage barriers (32), each leakage barrier (32) being joined to the inner main surface (74) of the main portion (7) along a leakage barrier joint (32') extending in said length direction (L) at least along said crotch portion (14), said leakage barrier (32) extending from said leakage barrier joint (32') in the height and/or width direction (H, W) along a leakage barrier width to a leakage barrier edge (32"),

   wherein a leakage barrier active region is provided at least in said crotch portion (14) of the main portion (7), wherein each leakage barrier (32) extends freely from said leakage barrier joint (32') along said leakage barrier width to a free leakage barrier edge (32"),
   wherein said free leakage barrier edge (32") comprises a leakage barrier elastic member (33) to provide elasticity generally along the length direction (L) of said free leakage barrier edge (32"),
   wherein said pocket member (20) is arranged in a non-overlapping relationship with said leakage barrier elastic member (33) as seen in the height direction (H) of the article (1).

21. The absorbent article according to claim 20, wherein there is a distance (De) in the length direction (L) between said pocket front edge (26) and said leakage barrier elastic member (33), said distance being at least 10mm.

22. The absorbent article according to any one of the claims 20 to 21, wherein said pocket member (20) is arranged in a non-overlapping relationship with said leakage barrier active region, as seen in the height direction H.

23. The absorbent article according to any one of the claims 20 to 22, wherein there is a distance (Da) in the length direction L between said pocket front edge (26) and said leakage barrier active region.

24. The absorbent article according to any one of the claims 20 to 23, wherein a leakage barrier fastening region is provided, wherein said leakage barrier (32) is fastened to said inner main surface (74) of said main portion (7) at a leakage barrier fastening element (34) between said leakage barrier joint (32') and said leakage barrier edge (32"), and/or at said leakage barrier edge (32"), such that said leakage barrier (32) extends freely from said leg fastening element (34) in the height and/or width direction along a leakage barrier unfastened width being less than said leakage barrier width.

25. The absorbent article according to claim 24, wherein said pocket member (20) is arranged in a partly overlapping relationship with said leakage barrier fastening region as seen in the height direction.

26. The absorbent article according to claim 25, wherein said leakage barrier unfastened width is less than 50% of said leakage barrier width, such as less than 20% of said leakage barrier width, such as less than 10% of said leakage barrier width, for example 0.


**Patentansprüche**

1. Absorbierender Artikel (1), umfassend einen Hauptabschnitt (7), der sich in einer Längsrichtung (L) entlang einer mittleren Längsachse (Lc) zwischen einer vorderen Kante (71) und einer hinteren Kante (72) des absorbierenden Artikels (1), und in einer Breitenrichtung (W) entlang einer mittleren Breitenachse (Wc), senkrecht zu der mittleren Längsachse (L) zwischen einem Paar von seitlichen Kanten (73) des absorbierenden Artikels (1) erstreckt, und sich in einer Höhenrichtung (H) senkrecht zu der Breiten- und der Längsrichtung (L, W) erstreckt,

wobei der Hauptabschnitt (7) eine innere Hauptfläche (74), die sich hauptsächlich entlang der Breiten- und der Längsrichtung (W, L) erstreckt, um beim Tragen des Artikels zum Träger hin gerichtet zu sein, und eine äußere Hauptfläche (75), die sich hauptsächlich entlang der Breiten- und der Längsrichtung (W, L) erstreckt, um beim Tragen des Artikels vom Träger weg gerichtet zu sein, definiert;
der Hauptabschnitt definierend
einen vorderen Bereich (10), umfassend die vordere Kante (71), und einen hinteren Bereich (72), umfassend die hintere Kante (72), und einen Schrittbereich (14), der sich zwischen dem vorderen und dem hinteren Bereich (10, 12) befindet, wobei der vordere Bereich (10) und der hintere Bereich (12) so angepasst sind, dass die vordere Kante (71) und die hintere Kante (72) einen Teil einer Taillenkante (16) bilden, die beim Tragen des Artikels (1) die Taille eines Benutzers umgibt,
wobei der Artikel (1) weiter ein Taschenelement (20) umfasst, das sich entlang einer Taschenlänge (Lp) in der Längsrichtung (L) zwischen einer hinteren Taschenkante (28) und einer vorderen Taschenkante (26) und entlang einer Taschenbreite (Wp) in der Breitenrichtung (W) zwischen einem Paar von seitlichen Taschenkanten (29) erstreckt,
wobei das Taschenelement (20) an dem hinteren Bereich (12) des Hauptabschnitts (7) durch ein hinteres Anbringungselement (27r), das sich entlang der hinteren Taschenkante (28) erstreckt, und durch seitliche Anbringungselemente (27s) angebracht ist, wobei sich jedes seitliche Anbringungselement (27s) entlang einer seitlichen Taschenkante (29) erstreckt, sodass das hintere und die seitlichen Anbringungselemente (27r, 27s) eine geschlossene Fäkalienbegrenzung zwischen dem Taschenelement (20) und dem Hauptabschnitt (7) des absorbierenden Artikels (1) mit einer Öffnung an der vorderen Taschenkante (26) in Richtung des Schrittbereichs (14) des Hauptabschnitts (7) bilden,
**gekennzeichnet durch**
das Taschenelement (20) umfassend eine Vielzahl von elastischen Elementen (25), die dem Taschenelement (20) elastische Eigenschaften entlang der Breitenrichtung (W) bereitstellen,
Verteilt sein der elastischen Elemente (25) über die Taschenlänge (Lp) des Taschenelements (20), um
eine erste elastische Zone (22), die sich von der hinteren Taschenkante (28) entlang der Längsrichtung (L) erstreckt, und
eine zweite elastische Zone (24), die sich von der vorderen Taschenkante (26) entlang der Längsrichtung (L) erstreckt, und eine Zwischenzone (21), die sich in der Längsrichtung (L) von der ersten elastischen Zone (22) zu der zweiten elastischen Zone (24) und über mindestens 20 % der Taschenlänge (Lp) erstreckt, zu bilden, und

eine durchschnittliche Verteilung der elastischen Elemente in der Zwischenzone, die weniger als 0,5 elastische Elemente pro cm beträgt, wie in der Längsrichtung (L) gesehen, und eine durchschnittliche Verteilung der elastischen Elemente (25) in jeder von der ersten und der zweiten elastischen Zone (22, 24), die größer ist als die durchschnittliche Verteilung von elastischen Elementen in der Zwischenzone (21), wie in der Längsrichtung (L) gesehen.

2. Einwegartikel nach Anspruch 1, wobei sich die Zwischenzone (21) in der Längsrichtung (L) über 80 % oder weniger der Taschenlänge (Lp) erstreckt.

3. Einwegartikel nach Anspruch 1 oder 2, wobei sich jede von der ersten und der zweiten elastischen Zone (22, 24) über mindestens 10 % der Taschenlänge (Lp) erstreckt, wie in der Längsrichtung (L) gesehen.

4. Absorbierender Artikel nach einem der vorstehenden Ansprüche, wobei in jeder von der ersten und/oder der zweiten elastischen Zone (22, 24) eine durchschnittliche Verteilung der elastischen Elemente (25) größer als 0,5 Elemente/cm ist, wie beispielsweise größer als 1 Element/cm, wie beispielsweise von 1 bis 3 Elemente/cm, wie in der Längsrichtung (L) gesehen.

5. Absorbierender Artikel nach einem der vorstehenden Ansprüche, wobei in jeder ersten und/oder zweiten elastischen Zone ein Abstand, wie in der Längsrichtung L gesehen, zwischen jedem Paar von zwei benachbarten elastischen Elementen (25) mindestens 3 mm beträgt, wie beispielsweise innerhalb des Bereichs von 3 bis 7 mm liegt, wie in der Längsrichtung (L) gesehen.

6. Absorbierender Artikel nach einem der vorstehenden Ansprüche, wobei die elastischen Elemente (25) elastische Fäden sind.

7. Absorbierender Artikel nach einem der vorstehenden Ansprüche, wobei eines oder mehrere, vorzugsweise alle, der elastischen Elemente (25) in der ersten und der zweiten elastischen Zone (22,24) eine Elastizität innerhalb des Bereichs von 100 % bis 250 % aufweisen.

8. Absorbierender Artikel nach einem der vorstehenden Ansprüche, wobei die erste elastische Zone (22) benachbart zur hinteren Kante (72) des Hauptabschnitts (7) angeordnet ist, um ein hinteres Taillengummiband zu bilden, das beim Tragen des Artikels (1) zur Elastizität des hinteren Bereichs (12) des Hauptabschnitts (7) um die Taille des Benutzers herum beiträgt.

9. Absorbierender Artikel nach einem der vorstehenden Ansprüche, wobei der hintere Bereich (12) des Hauptabschnitts (7) frei von elastischen Elementen ist, die Elastizität in der Breitenrichtung (W) bereitstellen.

10. Absorbierender Artikel nach einem der vorstehenden Ansprüche, wobei der Hauptabschnitt (7) frei von elastischen Elementen ist, die Elastizität in der Breitenrichtung (W) bereitstellen.

11. Absorbierender Artikel nach einem der vorstehenden Ansprüche, wobei das hintere Anbringungselement (27r) mindestens teilweise, vorzugsweise vollständig, die erste elastische Zone (22) überlappt, wie entlang der Höhenrichtung (H) gesehen.

12. Absorbierender Artikel nach einem der vorstehenden Ansprüche, wobei das Taschenelement (20) ein Blattmaterial (23), wie beispielsweise ein Vliesmaterial, umfasst,

wobei das Taschenelement (20) zwei Lagen des Blattmaterials (23) umfasst, wobei die elastischen Elemente (25) zwischen den beiden Lagen des Blattmaterials (23) positioniert sind.

13. Absorbierender Artikel nach Anspruch 12, wobei die beiden Lagen durch ein kontinuierliches Stück Blattmaterial (23), das um die elastischen Elemente (25) herum gefaltet ist, gebildet sind und durch eine Verbindung (40) verbunden sind, um das Taschenelement (20) zu bilden.

14. Absorbierender Artikel nach einem der Ansprüche 12 bis 13, wobei das Blattmaterial (23) ein Flächengewicht innerhalb des Bereichs von 10 gsm (Gramm pro Quadratmeter) bis 60 gsm aufweist, wie beispielsweise, wobei das Blattmaterial beispielsweise ein Vliesmaterial ist, das ein Flächengewicht innerhalb des Bereichs von 10 gsm bis 30 gsm aufweist.

15. Absorbierender Artikel nach einem der Ansprüche 12 bis 14, wobei das Blattmaterial (23) eine Luftdurchlässigkeit von mindestens 1000 l/m2/s, wie beispielsweise mindestens 2000 l/m2/s, und/oder eine Materialwasserdampfübertragungsrate von mindestens 2000 g/m2/24 h, wie beispielsweise mindestens 4000 g/m2/24 h, aufweist.

16. Absorbierender Artikel nach einem der Ansprüche 12 bis 15, wobei das Blattmaterial (23) ein Vliesmaterial ist, das Fasern umfasst, die aus einer oder mehreren Komponenten bestehen, wobei die Komponenten Polymerkomponenten wie beispielsweise PP oder PE sind, wie beispielsweise PP-PE-Fasern und/oder PP-PP-Fasern.

**17.** Absorbierender Artikel nach einem der vorstehenden Ansprüche, wobei der Hauptabschnitt (7) einen absorbierenden Kern (30) umfasst, der sich mindestens im Schrittbereich (14) des Hauptabschnitts (7) erstreckt,

wobei der Hauptabschnitt (7) ein Deckblatt (3), das mindestens einen Teil der inneren Hauptfläche (74) bildet, und ein Rückblatt (4), das mindestens einen Teil der äußeren Hauptfläche (75) bildet, umfasst, wobei der absorbierende Kern (30) zwischen dem Deckblatt (3) und dem Rückblatt (4) angeordnet ist,

wobei das Taschenelement (20) in Bezug auf den absorbierenden Kern (30) in einer nicht überlappenden Beziehung angeordnet ist, wie in der Höhenrichtung (H) gesehen.

**18.** Absorbierender Artikel nach Anspruch 17, wobei ein Abstand (De) in der Längsrichtung (L) zwischen der vorderen Taschenkante (26) und dem absorbierenden Kern (30) 5 mm oder mehr beträgt.

**19.** Absorbierender Artikel nach einem der Ansprüche 17 bis 18, wobei jedes einzelne der seitlichen Anbringungselemente (27s) außerhalb des absorbierenden Kerns (30) angeordnet ist, wie entlang der Breitenrichtung (W) gesehen.

**20.** Absorbierender Artikel nach einem der vorstehenden Ansprüche, wobei der absorbierende Artikel (1) weiter ein Paar von Auslaufsperren (32) umfasst, wobei jede Auslaufsperre (32) mit der inneren Hauptfläche (74) des Hauptabschnitts (7) entlang einer Auslaufsperrenverbindung (32'), die sich in der Längsrichtung (L) mindestens entlang des Schrittabschnitts (14) erstreckt, verbunden ist, wobei sich die Auslaufsperre (32) von der Auslaufsperrenverbindung (32') in der Höhen- und/oder der Breitenrichtung (H, W) entlang einer Auslaufsperrenbreite zu einer Auslaufsperrenkante (32") erstreckt,

wobei ein aktiver Auslaufsperrenbereich mindestens im Schrittabschnitt (14) des Hauptabschnitts (7) bereitgestellt ist, wobei sich jede Auslaufsperre (32) frei von der Auslaufsperrenverbindung (32') entlang der Auslaufsperrenbreite zu einer freien Auslaufsperrenkante (32") erstreckt,

wobei die freie Auslaufsperrenkante (32") ein elastisches Auslaufsperrenelement (33) umfasst, um im Allgemeinen entlang der Längsrichtung (L) der freien Auslaufsperrenkante (32") Elastizität bereitzustellen,

wobei das Taschenelement (20) in einer nicht überlappenden Beziehung mit dem elastischen Auslaufsperrenelement (33) angeordnet ist, wie in der Höhenrichtung (H) des Artikels (1) gesehen.

**21.** Absorbierender Artikel nach Anspruch 20, wobei es in der Längsrichtung (L) einen Abstand (De) zwischen der vorderen Taschenkante (26) und dem elastischen Auslaufsperrenelement (33) gibt, wobei dieser Abstand mindestens 10 mm beträgt.

**22.** Absorbierender Artikel nach einem der Ansprüche 20 bis 21, wobei das Taschenelement (20) in einer nicht überlappenden Beziehung mit dem aktiven Auslaufsperrenbereich angeordnet ist, wie in der Höhenrichtung H gesehen.

**23.** Absorbierender Artikel nach einem der Ansprüche 20 bis 22, wobei es in der Längsrichtung L einen Abstand (Da) zwischen der vorderen Taschenkante (26) und dem aktiven Auslaufsperrenbereich gibt.

**24.** Absorbierender Artikel nach einem der Ansprüche 20 bis 23, wobei ein Auslaufsperrenbefestigungsbereich bereitgestellt ist, wobei die Auslaufsperre (32) an der inneren Hauptfläche (74) des Hauptabschnitts (7) an einem Auslaufsperrenbefestigungselement (34) zwischen der Auslaufsperrenverbindung (32') und der Auslaufsperrenkante (32") und/oder an der Auslaufsperrenkante (32") befestigt ist, sodass sich die Auslaufsperre (32) in der Höhen- und/oder der Breitenrichtung entlang einer nicht befestigten Auslaufsperrenbreite, die geringer ist als die Auslaufsperrenbreite, frei von dem Beinbefestigungselement (34) erstreckt.

**25.** Absorbierender Artikel nach Anspruch 24, wobei das Taschenelement (20) in einer teilweise überlappenden Beziehung mit dem Auslaufsperrenbefestigungsbereich angeordnet ist, wie in der Höhenrichtung gesehen.

**26.** Absorbierender Artikel nach Anspruch 25, wobei die nicht befestigte Auslaufsperrenbreite weniger als 50 % der Auslaufsperrenbreite, wie beispielsweise weniger als 20 % der Auslaufsperrenbreite, wie beispielsweise weniger als 10 % der Auslaufsperrenbreite, zum Beispiel 0 beträgt.

**Revendications**

**1.** Un article absorbant (1) comprenant une partie principale (7) s'étendant dans une direction de longueur (L) le long d'un axe de longueur central (Lc) entre un bord avant (71) et un bord arrière (72) dudit article absorbant (1), et dans une direction de largeur (W) le long d'un axe de largeur central (Wc), perpendiculaire audit axe de longueur central (L) entre une paire de bords latéraux (73) dudit article absorbant (1), et s'étendant dans une direction de hauteur (H) perpendiculaire auxdites directions de largeur et de longueur (L, W),

la partie principale (7) définissant une surface principale intérieure (74) s'étendant principalement le long desdites directions de largeur et longueur (W, L) à diriger vers le porteur lorsque l'article est porté, et une surface extérieure principale (75) s'étendant principalement le long desdites directions de largeur et longueur (W, L) à diriger à l'écart du porteur lorsque l'article est porté ;

la partie principale définissant

une région avant (10) comprenant ledit bord avant (71) et une région arrière (72) comprenant ledit bord arrière (72), et une région d'entrejambe (14) située entre les régions avant et arrière (10, 12), la région avant (10) et la région arrière (12) étant adaptées de sorte que ledit bord avant (71) et ledit bord arrière (72) fassent partie d'un bord de taille (16) circonférant la taille de l'utilisateur lorsque l'article (1) est porté, l'article (1) comprenant en outre un membre de poche (20) s'étendant le long d'une longueur de poche (Lp) dans ladite direction de longueur (L) entre un bord arrière de poche (28) et un bord avant de poche (26), et le long d'une largeur de poche (Wp) dans ladite direction de largeur (W) entre une paire de bords latéraux de poche (29), où ledit membre de poche (20) est fixé à la région arrière (12) de la partie principale (7) par un élément d'attache arrière (27r) s'étendant le long du bord arrière de poche (28) et par des éléments d'attache latéraux (27s), chaque élément d'attache latéral (27s) s'étendant le long d'un bord latéral de poche (29) de sorte que lesdits éléments d'attache arrière et latéraux (27r, 27s) forment une limite fécale fermée entre ledit membre de poche (20) et ladite partie principale (7) dudit article absorbant (1) avec une ouverture audit bord avant de poche (26) vers la région d'entrejambe (14) de ladite partie principale (7),

**caractérisé par**

ledit membre de poche (20) comprenant une pluralité de membres élastiques (25) fournissant audit membre de poche (20) des caractéristiques élastiques le long de ladite direction de largeur (W),

lesdits membres élastiques (25) étant répartis sur la longueur de poche (Lp) dudit membre de poche (20) afin de former

une première zone élastique (22) s'étendant depuis ledit bord arrière de poche (28) le long de la direction de longueur (L) et

une seconde zone élastique (24) s'étendant depuis ledit bord avant de poche (26) le long de la direction de longueur (L), et une zone intermédiaire (21) s'étendant dans ladite direction de longueur (L) depuis ladite première zone élastique (22) à ladite seconde zone élastique (24) et sur au moins 20 % de la longueur de poche (Lp), et

une distribution moyenne desdits membres élastiques dans ladite zone intermédiaire est inférieure à 0,5 membres élastiques par cm comme observé dans la direction de longueur (L), et

une distribution moyenne desdits membres élastiques (25) dans chacune desdites première et seconde zones élastiques (22, 24) est supérieure à ladite distribution moyenne de membres élastiques dans ladite zone intermédiaire (21), comme observé dans la direction de longueur (L).

2. L'article jetable selon la revendication 1, dans lequel ladite zone intermédiaire (21) s'étend dans ladite direction de longueur (L) sur 80 % ou moins de la longueur de poche (Lp).

3. L'article jetable selon la revendication 1 ou 2, dans lequel chacune des première et deuxième zones élastiques (22, 24) s'étend sur au moins 10 % de la longueur de poche (Lp) comme observé dans la direction de longueur (L).

4. L'article absorbant selon l'une des revendications précédentes, dans lequel, dans chacune desdites première et/ou seconde zones élastiques (22, 24), une distribution moyenne desdits membres élastiques (25) est supérieure à 0,5 membre/cm, par exemple supérieure à 1 membre/cm, comme de 1 à 3 membres/cm comme observé dans la direction de longueur (L).

5. L'article absorbant selon l'une des revendications précédentes, dans lequel, dans chaque première et/ou deuxième zone élastique, une distance telle que vue dans la direction de longueur L entre chaque paire de deux membres élastiques voisins (25) est d'au moins 3 mm, comme dans la plage de 3 à 7 mm, comme observé dans la direction de longueur (L).

6. L'article absorbant selon l'une des revendications précédentes, dans lequel lesdits membres élastiques (25) sont des fils élastiques.

7. L'article absorbant selon l'une des revendications précédentes, dans lequel un ou plusieurs, de préférence tous les membres élastiques (25) dans lesdites première et deuxième zones élastiques (22, 24) ont une élasticité allant de 100 % à 250 %.

8. L'article absorbant selon l'une des revendications précédentes, dans lequel ladite première zone élastique (22) est disposée à côté dudit bord arrière (72) de la partie principale (7), de sorte à former un élastique arrière de taille, contribuant à l'élasticité de la région arrière (12) de ladite partie principale (7) autour de la taille de l'utilisateur lorsque ledit article (1) est porté.

9. L'article absorbant selon l'une des revendications précédentes, dans lequel ladite région arrière (12) de ladite partie principale (7) est libre de membres élastiques fournissant de l'élasticité dans la direction de largeur (W).

10. L'article absorbant selon l'une des revendications précédentes, dans lequel la partie principale (7) est libre de membres élastiques fournissant de l'élasticité dans la direction de largeur (W).

11. L'article absorbant selon l'une des revendications précédentes, dans lequel ledit élément d'attache arrière (27r) chevauche au moins partiellement, de préférence complètement, la première zone élastique (22) comme observé le long de la direction de hauteur (H).

12. L'article absorbant selon l'une des revendications précédentes, dans lequel le membre de poche (20) comprend un matériau en feuille (23), tel qu'un matériau non tissé, où ledit membre de poche (20) comprend deux couches de matériau en feuille (23), où lesdits membres élastiques (25) sont positionnés entre lesdites deux couches de matériau en feuille (23).

13. L'article absorbant selon la revendication 12, dans lequel lesdites deux couches sont formées par une pièce continue de matériau en feuille (23) pliée autour desdits membres élastiques (25), et reliée par un joint (40) pour former ledit membre de poche (20).

14. L'article absorbant selon l'une des revendications 12 à 13, dans lequel ledit matériau en feuille (23) a un poids de base dans la plage de 10 gsm à 60 gsm, par exemple ledit matériau en feuille étant un matériau non tissé ayant un poids de base dans la plage de 10 gsm à 30 gsm.

15. L'article absorbant selon l'une des revendications 12 à 14, dans lequel ledit matériau en feuille (23) a une perméabilité à l'air d'au moins 1000 l/m2/s, comme au moins 2000 l/m2/s et/ou un taux de transmission de vapeur d'eau de matériau d'au moins 2000 g/m2/24h, comme au moins 4000 g/m2/24h.

16. L'article absorbant selon l'une des revendications 12 à 15, dans lequel le matériau en feuille (23) est un matériau non tissé comprenant des fibres composées d'un ou plusieurs composants, les composants étant des composants polymères tels que le PP ou le PE, tels que les fibres PP-PE et/ou des fibres PP-PP.

17. L'article absorbant selon l'une des revendications précédentes dans lequel ladite partie principale (7) comprend un noyau absorbant (30) s'étendant au moins dans la région d'entrejambe (14) de la partie principale (7),

   où ladite partie principale (7) comprend une feuille supérieure (3) formant au moins une partie de ladite surface intérieure principale (74), et une feuille arrière (4) formant au moins une partie de ladite surface principale extérieure (75), ledit noyau absorbant (30) étant disposé entre ladite feuille supérieure (3) et ladite feuille arrière (4),
   où ledit membre de poche (20) est disposé dans une relation non chevauchante par rapport audit noyau absorbant (30) comme observé dans la direction de hauteur (H).

18. L'article absorbant selon la revendication 17, dans lequel une distance (Dc) dans la direction de longueur (L) entre ledit bord avant de poche (26) et ledit noyau absorbant (30) est de 5 mm ou plus.

19. L'article absorbant selon l'une des revendications 17 à 18, dans lequel chacun desdits éléments d'attache latéraux (27s) est disposé vers l'extérieur dudit noyau absorbant (30), comme observé le long de la direction de largeur (W).

20. L'article absorbant selon l'une des revendications précédentes, dans lequel ledit article absorbant (1) comprend en outre une paire d'obstacles à fuite (32), chaque obstacle à fuite (32) étant relié à la surface intérieure principale (74) de la partie principale (7) le long d'un joint d'obstacle à fuite (32') s'étendant dans ladite direction de longueur (L) au moins le long de ladite partie d'entrejambe (14), ledit obstacle à fuite (32) s'étendant depuis ledit joint d'obstacle à fuite (32') dans la direction de hauteur et/ou de largeur (H, W) le long d'une largeur d'obstacle à fuite jusqu'à un bord d'obstacle à fuite (32"),

   où une zone active d'obstacle à fuite est prévue au moins dans ladite partie d'entrejambe (14) de la partie principale (7), où chaque obstacle à fuite (32) s'étend librement depuis ledit joint d'obstacle à fuite (32') le long de ladite largeur d'obstacle à fuite jusqu'à un bord d'obstacle à fuite libre (32"),
   où ledit bord d'obstacle à fuite libre (32") comprend un membre élastique d'obstacle à fuite (33) pour fournir une élasticité généralement le long de la direction de longueur (L) dudit bord d'obstacle à fuite libre (32"),

où ledit membre de poche (20) est disposé en relation non chevauchante avec ledit membre élastique d'obstacle à fuite (33), comme observé dans la direction de hauteur (H) de l'article (1).

21. L'article absorbant selon la revendication 20, dans lequel il existe une distance (De) dans la direction de longueur (L) entre le bord avant de poche (26) et le membre élastique d'obstacle à fuite (33), ladite distance étant d'au moins 10 mm.

22. L'article absorbant selon l'une des revendications 20 à 21, dans lequel ledit membre de poche (20) est disposé en relation non chevauchante avec la zone active d'obstacle à fuite, comme observé dans la direction de hauteur H.

23. L'article absorbant selon l'une des revendications 20 à 22, dans lequel il existe une distance (Da) dans la direction de longueur L entre le bord avant de poche (26) et la zone active d'obstacle à fuite.

24. L'article absorbant selon l'une des revendications 20 à 23, dans lequel une région de fixation d'obstacle à fuite est prévue, où ledit obstacle à fuite (32) est fixé à ladite surface intérieure principale (74) de ladite partie principale (7) à un élément de fixation d'obstacle à fuite (34) entre ledit joint d'obstacle à fuite (32') et ledit bord d'obstacle à fuite (32"), et/ou audit bord d'obstacle à fuite (32"), de sorte que ledit obstacle à fuite (32) s'étend librement depuis ledit élément de fixation de jambe (34) dans la direction de hauteur et/ou de largeur le long d'une largeur non fixée d'obstacle à fuite qui est inférieure à ladite largeur d'obstacle à fuite.

25. L'article absorbant selon la revendication 24, dans lequel ledit membre de poche (20) est disposé en une relation partiellement chevauchante avec la région de fixation d'obstacle à fuite telle que vue dans la direction de hauteur.

26. L'article absorbant selon la revendication 25, selon lequel ladite largeur non fixée d'obstacle à fuite est inférieure à 50 % de ladite largeur d'obstacle à fuite, par exemple moins de 20 % de ladite largeur d'obstacle à fuite, par exemple moins de 10 % de ladite largeur d'obstacle à fuite, par exemple 0.

Fig. 1

Fig. 2

Fig. 3

Fig. 4

EP 4 440 526 B1

Fig. 5

Fig. 6

**REFERENCES CITED IN THE DESCRIPTION**

**Patent documents cited in the description**

- US 10470943 B2